# EUROPEAN PATENT APPLICATION

(11) **EP 4 640 075 A1**
(43) Date of publication of application: **29.10.2025**
(21) Application number: 24171606.7
(22) Date of filing: 22.04.2024
(51) Int. Cl.: A24B 15/16, A24B 15/30, A61K 9/107

(54) **ORAL POUCHED PRODUCT WITH SELF-EMULSIFYING DELIVERY SYSTEM**

(71) Applicant: Nicoventures Trading Limited, London WC2R 3LA (GB)
(72) Inventor: Daniel, Michael S., North Carolina (US); Roohinejad, Shahin, North Carolina (US)
(74) Representative: Newcombe, Christopher David

(57) **Abstract**

The disclosure provides a pouched oral product including a pouch and a composition enclosed therein. The composition includes a self-emulsifying delivery system (SEDS) component configured to provide droplets in the mouth of a user of the pouched oral product. The SEDS component includes a flavoring agent, an active ingredient, or a combination thereof; at least one solubilizer; at least one emulsifying agent; and optionally, an antioxidant, a lipid component, or both.

## Description

### FIELD OF THE DISCLOSURE

The present disclosure relates to an oral product. In particular, the present disclosure relates to products intended for human consumption. The products are configured for oral use and deliver substances such as flavors and/or active ingredients during use.

### BACKGROUND

There are many categories of products intended for oral use and enjoyment. For example, oral tobacco products containing nicotine, which is known to have both stimulant and anxiolytic properties, have been available for many years. Conventional formats for so-called "smokeless" tobacco products include moist snuff, snus, and chewing tobacco, which are typically formed almost entirely of particulate, granular, or shredded tobacco, and which are either portioned by the user or presented to the user in individual portions, such as in single-use pouches or sachets. See for example, the types of smokeless tobacco formulations, ingredients, and processing methodologies set forth in US Pat. Nos. 6,668,839 to Williams; 6,834,654 to Williams; 6,953,040 to Atchley et al.; 7,032,601 to Atchley et al.; and 7,694,686 to Atchley et al.; 7,810,507 to Dube et al.; 7,819,124 to Strickland et al.; 7,861,728 to Holton, Jr. et al.; 7,901,512 to Quinter et al.; 8,627,828 to Strickland et al.; 11,246,334 to Atchley, each of which is incorporated herein by reference.

In addition, traditional tobacco materials and non-tobacco materials have been combined with other ingredients to form product formats distinct from traditional smokeless products, with example formats including lozenges, pastilles, gels, and the like. See, for example, the types of products described in US Patent App. Pub. Nos. 2008/0196730 to Engstrom et al.; 2008/0305216 to Crawford et al.; 2009/0293889 to Kumar et al.; 2010/0291245 to Gao et al; 2011/0139164 to Mua et al.; 2012/0037175 to Cantrell et al.; 2012/0055494 to Hunt et al.; 2012/0138073 to Cantrell et al.; 2012/0138074 to Cantrell et al.; 2013/0074855 to Holton, Jr.; 2013/0074856 to Holton, Jr.; 2013/0152953 to Mua et al.; 2013/0274296 to Jackson et al.; 2015/0068545 to Moldoveanu et al.; 2015/0101627 to Marshall et al.; and 2015/0230515 to Lampe et al., each of which is incorporated herein by reference.

There is continuing interest in the development of new types of oral products that deliver advantageous sensorial or biological activity. Such products typically contain flavorants and/or active ingredients such as nicotine, caffeine, botanicals, or cannabidiol. The format of such products can vary, and include pouched products containing a powdered or granular composition, lozenges, pastilles, liquids, gels, emulsions, meltable compositions, and the like. See, for example, the types of products described in US Patent App. Pub. Nos. 2022/0160675 to Gerardi et al.; 2022/0071984 to Poole et al.; 2021/0378948 to Gerardi et al.; 2021/0330590 to Hutchens et al.; 2021/0186081 to Gerardi et al.; 2021/0177754 to Keller et al; 2021/0177043 to Gerardi et al.; 2021/0177038 to Gerardi et al.; 2021/0169867 to Holton, Jr. et al.; 2021/0169792 to Holton, Jr. et al.; 2021/0169132 to Holton, Jr. et al.; 2021/0169121 to St. Charles, and 2021/0169122 to St. Charles, each of which is incorporated herein by reference.

### BRIEF SUMMARY

In accordance with some embodiments described herein, there is provided an oral product comprising a lipophilic active ingredient and a self-emulsifying delivery system (SEDS). The SEDS is configured to provide droplets comprising the lipophilic active ingredient. Particularly, the SEDS is configured to form a nanoemulsion (i.e., micelles) in the mouth and gastrointestinal tract of the user upon use of the oral product.

Accordingly, in one aspect is provided a pouched oral product comprising a pouch and a composition enclosed therein, the composition comprising a self-emulsifying delivery system (SEDS) component the SEDS component comprising: a flavoring agent, an active ingredient, or a combination thereof; at least one solubilizer; at least one emulsifying agent; and optionally, an antioxidant, a lipid component, or both an antioxidant and a lipid component.

In some embodiments, the active ingredient comprises one or more botanical materials, stimulants, nicotine components, amino acids, vitamins, cannabinoids, cannabimimetics, terpenes, nutraceuticals, pharmaceutical agents, or combinations thereof.

In some embodiments, the flavoring agent comprises one or more aldehydes, ketones, esters, terpenes, terpenoids, or a combination thereof.

In some embodiments, the at least one solubilizer comprises a glycerol monoester of a fatty acid.

In some embodiments, the at least one emulsifying agent has a hydrophilic-lipophilic balance (HLB) value in a range from about 3 to about 5. In some embodiments, the at least one emulsifying agent comprises polyethoxylated castor oil, a polyethylene glycol monoester of a fatty acid, a polyethylene glycol diester of a fatty acid, or a combination thereof.

In some embodiments, the antioxidant is a tocopherol, BHT, a TBHQ, vitamin E or a derivative thereof, citric acid, a fatty acid ester of vitamin C, or a combination thereof.

In some embodiments, the lipid component is a natural, food-grade oil. In some embodiments, the lipid component is olive oil.

In some embodiments, the SEDS component comprises:
from about 40 to about 60% by weight of polyethoxylated castor oil;
from about 17 to about 25% by weight of linoleic acid monoglyceryl ester;
from about 17 to about 25% by weight of a mixture of polyethylene glycol 6 mono- and diesters of oleic acid; and
from about 0.4 to about 0.6% by weight of tocopherols;
wherein each amount by weight is based on the total weight of the SEDS component.

In some embodiments, the SEDS component is at least partially in form of a pre-emulsion comprising micelles having an average particle size in a range from about 100 to about 300 nm.

In some embodiments, the SEDS component is configured to provide droplets in the mouth of a user of the pouched oral product upon use of the oral product.

In some embodiments, the composition further comprises: a filler; a carrier material; optionally, a further active ingredient, a further flavoring agent, or a combination thereof; and optionally, one or more sweeteners, one or more humectants, one or more binding agents, one or more salts, one or more buffering agents, or a combination thereof.

In some embodiments, the oral product comprises the SEDS component in an amount by weight from about 2 to about 5%, based on the total weight of the oral product.

In some embodiments, the SEDS component is adsorbed in or on the carrier material. In some embodiments, the carrier material is silica.

In some embodiments, the filler comprises a cellulose material. In some embodiments, the cellulose material comprises microcrystalline cellulose.

In some embodiments, the oral product comprises:
from about 10 to about 50% of the filler; and
from about 5 to about 60% by weight of water, based on the total weight of the oral product.

In some embodiments, the further active ingredient comprises a nicotine component, botanical materials, stimulants, amino acids, vitamins, antioxidants, cannabinoids, cannabimimetics, terpenes, nutraceuticals, pharmaceutical agents, or combinations thereof. In some embodiments, the further active ingredient comprises a nicotine component.

In some embodiments, the composition further comprises one or more organic acids, an alkali metal salt of the one or more organic acids, or both.

In some embodiments, the one or more organic acids or alkali metal salt thereof is present in the composition in an amount by weight of from about 0.1 to about 10%, or from about 0.1 to about 0.5%, based on the total weight of the composition.

In some embodiments, the one or more organic acids is citric acid, malic acid, tartaric acid, octanoic acid, benzoic acid, a toluic acid, salicylic acid, or a combination thereof.

In some embodiments, at least a portion of the organic acid, the alkali metal salt of the organic acid, or both are associated with at least a portion of the nicotine component in the form of an ion pair.

In some embodiments, the SEDS component provides micelles comprising the flavoring agent, the active ingredient, or both, when dispersed in water at a ratio of about 100: 1 water to SEDS component by weight.

In some embodiments, the micelles have a particle size D50 of about 60 nm.

In some embodiments, the SEDS component comprises a flavoring agent, and when subjected to mouth conditions in the mouth of a user, the oral product provides a duration of perceived flavor of up to about 45 minutes.

In another aspect is provided a method of preparing a pouched oral product comprising a self-emulsifying delivery system (SEDS) component comprising at least one solubilizer, at least one emulsifying agent, and optionally, an antioxidant, a lipid component, or both an antioxidant and a lipid component, the method comprising:
combining the at least one solubilizer, the at least one emulsifying agent, and optionally the antioxidant and/or lipid component to form the SEDS component;
contacting the SEDS component with a carrier material to form an adsorbed SEDS component;
combining the adsorbed SEDS component with:
   a filler;
   an active ingredient, a flavoring agent, or a combination thereof; and
   optionally, one or more sweeteners, one or more humectants, one or more binding agents, one or more salts, one or more buffering agents, or a combination thereof, to form a composition configured for oral use;
enclosing the composition in a pouch; and
adding an amount of water to the pouch.

In some embodiments, the at least one solubilizer comprises a glycerol monoester of a fatty acid.

In some embodiments, the at least one emulsifying agent has a hydrophilic-lipophilic balance (HLB) value in a range from about 3 to about 5. In some embodiments, the at least one emulsifying agent comprises polyethoxylated castor oil, a polyethylene glycol monoester of a fatty acid, a polyethylene glycol diester of a fatty acid, or a combination thereof.

In some embodiments, the antioxidant is a tocopherol, BHT, a fatty acid ester of vitamin C, or a combination thereof.

In some embodiments, the lipid component is a natural, food-grade oil. In some embodiments, the lipid component is olive oil.

In some embodiments, the filler comprises a cellulose material. In some embodiments, the cellulose material comprises microcrystalline cellulose.

In some embodiments, the carrier material is silica.

The disclosure includes, without limitations, the following embodiments.

Embodiment 1: A pouched oral product comprising a pouch and a composition enclosed therein, the composition comprising a self-emulsifying delivery system (SEDS) component the SEDS component comprising:
a flavoring agent, an active ingredient, or a combination thereof;
at least one solubilizer;
at least one emulsifying agent; and
optionally, an antioxidant, a lipid component, or both an antioxidant and a lipid component.

Embodiment 2: The oral product of embodiment 1, wherein the active ingredient comprises one or more botanical materials, stimulants, nicotine components, amino acids, vitamins, cannabinoids, cannabimimetics, terpenes, nutraceuticals, pharmaceutical agents, or combinations thereof.

Embodiment 3: The oral product of embodiment 1 or 2, wherein the flavoring agent comprises one or more aldehydes, ketones, esters, terpenes, terpenoids, or a combination thereof.

Embodiment 4: The oral product of any one of embodiments 1-3, wherein the at least one solubilizer comprises a glycerol monoester of a fatty acid.

Embodiment 5: The oral product of any one of embodiments 1-4, wherein the at least one emulsifying agent has a hydrophilic-lipophilic balance (HLB) value in a range from about 3 to about 5.

Embodiment 6: The oral product of any one of embodiments 1-5, wherein the at least one emulsifying agent comprises polyethoxylated castor oil, a polyethylene glycol monoester of a fatty acid, a polyethylene glycol diester of a fatty acid, or a combination thereof.

Embodiment 7: The oral product of any one of embodiments 1-6, wherein the antioxidant is a tocopherol, BHT, a TBHQ, vitamin E or a derivative thereof, citric acid, a fatty acid ester of vitamin C, or a combination thereof.

Embodiment 8: The oral product of any one of embodiments 1-7, wherein the lipid component is a natural, food-grade oil.

Embodiment 9: The oral product of embodiment 8, wherein the lipid component is olive oil.

Embodiment 10: The oral product of any one of embodiments 1-9, wherein the SEDS component comprises:
from about 40 to about 60% by weight of polyethoxylated castor oil;
from about 17 to about 25% by weight of linoleic acid monoglyceryl ester;
from about 17 to about 25% by weight of a mixture of polyethylene glycol 6 mono- and diesters of oleic acid;
from about 0.4 to about 0.6% by weight of tocopherols,
wherein each amount by weight is based on the total weight of the SEDS component.

Embodiment 11: The oral product of any one of embodiments 1-10, wherein the SEDS component is configured to provide droplets in the mouth of a user of the pouched oral product upon use of the oral product.

Embodiment 12: The oral product of any one of embodiments 1-11, wherein the SEDS component is at least partially in form of a pre-emulsion comprising micelles having an average particle size in a range from about 100 to about 300 nm.\

Embodiment 13: The oral product of any one of embodiments 1-12, wherein the composition further comprises:
a filler;
a carrier material;
a further active ingredient, a further flavoring agent, or a combination thereof; and
optionally, one or more sweeteners, one or more humectants, one or more binding agents, one or more salts, one or more buffering agents, or a combination thereof.

Embodiment 14: The oral product of any one of embodiments 1-13, comprising the SEDS component in an amount by weight from about 2 to about 5%, based on the total weight of the oral product.

Embodiment 15: The oral product of embodiment 13 or 14, wherein the SEDS component is adsorbed in or on the carrier material; optionally, wherein the carrier material is silica..

Embodiment 16: The oral product of any one of embodiments 13-15, wherein the filler comprises a cellulose material.

Embodiment 17: The oral product of embodiment 16, wherein the cellulose material comprises microcrystalline cellulose.

Embodiment 18: The oral product of any one of embodiments 13-17, comprising:
from about 10 to about 50% of the filler; and
from about 5 to about 60% by weight of water, based on the total weight of the oral product.

Embodiment 19: The oral product of any one of embodiments 13-18, wherein the further active ingredient comprises a nicotine component, botanical materials, stimulants, amino acids, vitamins, antioxidants, cannabinoids, cannabimimetics, terpenes, nutraceuticals, pharmaceutical agents, or combinations thereof.

Embodiment 20: The oral product of embodiment 19, wherein the further active ingredient comprises a nicotine component.

Embodiment 21: The oral product of embodiment 20, wherein the composition further comprises one or more organic acids, an alkali metal salt of the one or more organic acids, or both.

Embodiment 22: The oral product of embodiment 21, wherein the one or more organic acids or alkali metal salt thereof is present in the composition in an amount by weight of from about 0.1 to about 10%, or from about 0.1 to about 0.5%, based on the total weight of the composition.

Embodiment 23: The oral product of embodiment 21 or 22, wherein the one or more organic acids is citric acid, malic acid, tartaric acid, octanoic acid, benzoic acid, a toluic acid, salicylic acid, or a combination thereof.

Embodiment 24: The oral product of any one of embodiments 21-23, wherein at least a portion of the organic acid, the alkali metal salt of the organic acid, or both are associated with at least a portion of the nicotine component in the form of an ion pair.

Embodiment 25: The oral product of any one of embodiments 1-24, wherein the SEDS component provides micelles comprising the flavoring agent, the active ingredient, or both, when dispersed in water at a ratio of about 100: 1 water to SEDS component by weight.

Embodiment 26: The oral product of embodiment 25, wherein the micelles have a particle size D50 of about 60 nm.

Embodiment 27: The oral product of any one of embodiments 1-25, wherein the SEDS component comprises a flavoring agent, and wherein, when subjected to mouth conditions in the mouth of a user, provides a duration of perceived flavor of up to about 45 minutes.

Embodiment 28: A method of preparing a pouched oral product comprising a self-emulsifying delivery system (SEDS) component comprising at least one solubilizer, at least one emulsifying agent, and optionally, an antioxidant, a lipid component, or both an antioxidant and a lipid component, the method comprising:
combining the at least one solubilizer, the at least one emulsifying agent, and optionally, an antioxidant, a lipid component, or both an antioxidant and a lipid component, to form the SEDS component;
contacting the SEDS component with a carrier material to form an adsorbed SEDS component;
combining the adsorbed SEDS component with:
   a filler;
   an active ingredient, a flavoring agent, or a combination thereof; and
optionally, one or more sweeteners, one or more humectants, one or more binding agents, one or more salts, one or more buffering agents, or a combination thereof, to form a composition configured for oral use;
enclosing the composition in a pouch; and
adding an amount of water to the pouch.

Embodiment 29: The method of embodiment 28, wherein the at least one solubilizer comprises a glycerol monoester of a fatty acid.

Embodiment 30: The method of embodiment 28 or 29, wherein the at least one emulsifying agent has a hydrophilic-lipophilic balance (HLB) value in a range from about 3 to about 5.

Embodiment 31: The method of any one of embodiments 28-30, wherein the at least one emulsifying agent comprises polyethoxylated castor oil, a polyethylene glycol monoester of a fatty acid, a polyethylene glycol diester of a fatty acid, or a combination thereof.

Embodiment 32: The method of any one of embodiments 28-31, wherein the lipid component is a natural, food-grade oil.

Embodiment 33: The method of any one of embodiments 28-32, wherein the lipid component is olive oil.

Embodiment 34: The method of any one of embodiments 28-33, wherein the filler comprises a cellulose material.

Embodiment 35: The method of embodiment 34, wherein the cellulose material comprises microcrystalline cellulose.

Embodiment 36: The method of any one of embodiments 28-35, wherein the carrier material is silica.

These and other features, aspects, and advantages of the disclosure will be apparent from a reading of the following detailed description together with the accompanying drawings, which are briefly described below. The disclosure includes any combination of two, three, four, or more of the above-noted embodiments as well as combinations of any two, three, four, or more features or elements set forth in this disclosure, regardless of whether such features or elements are expressly combined in a specific embodiment description herein. This disclosure is intended to be read holistically such that any separable features or elements of the disclosure, in any of its various aspects and embodiments, should be viewed as intended to be combinable unless the context clearly dictates otherwise.

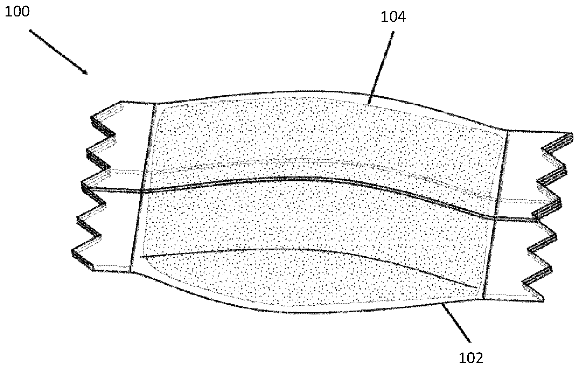

### BRIEF DESCRIPTION OF THE DRAWING

Having thus described aspects of the disclosure in the foregoing general terms, reference will now be made to the accompanying drawing, which is not necessarily drawn to scale. The drawing is exemplary only and should not be construed as limiting the disclosure. The drawing is a perspective view of a pouched product embodiment according to an example embodiment of the present disclosure including a pouch or fleece at least partially filled with an effervescent composition according to a non-limiting embodiment of the disclosure.

### DETAILED DESCRIPTION

The present disclosure will now be described more fully hereinafter with reference to example embodiments thereof. These example embodiments are described so that this disclosure will be thorough and complete, and will fully convey the scope of the disclosure to those skilled in the art. Indeed, the disclosure may be embodied in many different forms and should not be construed as limited to the embodiments set forth herein; rather, these embodiments are provided so that this disclosure will satisfy applicable legal requirements.

As used in this specification and the claims, the singular forms "a," "an," and "the" include plural referents unless the context clearly dictates otherwise.

The term "about" used throughout this specification is used to describe and account for small fluctuations. For example, the term "about" can refer to less than or equal to ±10%, such as less than or equal to ±5%, less than or equal to ±2%, less than or equal to ±1%, less than or equal to ±0.5%, less than or equal to ±0.2%, less than or equal to ±0.1% or less than or equal to ±0.05%. All numeric values herein are modified by the term "about," whether or not explicitly indicated. A value modified by the term "about" of course includes the specific value. For instance, "about 5.0" must include 5.0.

Reference to "dry weight percent" or "dry weight basis" refers to weight on the basis of dry ingredients (i.e., all ingredients except water). Reference to "wet weight" refers to the weight of the composition including water. Unless otherwise indicated, reference to "weight percent" of a composition reflects the total wet weight of the composition (i.e., including water).

The present disclosure provides an oral product comprising a pouch and a composition enclosed therein, the composition comprising a self-emulsifying delivery system (SEDS) component. The SEDS component is configured to provide droplets in the mouth of a user of the pouched oral product. The example individual components of the overall oral product, the composition therein, and components of the composition, are each described herein below.

### Self-Emulsifying Delivery System (SEDS) Component

The pouched product as disclosed herein comprises a pouch and a composition enclosed therein, the composition comprising a self-emulsifying delivery system (SEDS) component. As used herein, the terms "self-emulsifying delivery system component" or "SEDS component" refer to a component comprising a mixture of materials together which, when subject to moisture in the mouth of a user, in the gastrointestinal tract, or both, form an emulsion. Accordingly, a SEDS component may also be referred to as a "pre-emulsion." The SEDS component is configured to provide droplets in the mouth of a user of the pouched oral product, meaning when in contact with moisture (e.g., saliva and/or fluids in the digestive tract), the SEDS component forms an emulsion with said moisture in the form of droplets of varying sizes.

In some embodiments, the emulsion produced upon use of the product is a nanoemulsion. By "nanoemulsion" is meant a colloidal particulate system with particulates in the sub-micron size range, for example particulates having an average size of less than about 1,000 nm, such as from about 10 to about 1,000 nm, or from about 100 to about 300 nm. The particulates (referred to herein also as droplets) are generally spherical, and the surfaces of such droplets are generally hydrophilic, while the interiors are generally lipophilic.

The droplets may comprise, in the interior, the exterior, or both, one or more of flavoring agents, active ingredients, solubilizers, emulsifying agents, antioxidants, and lipids, each of which is further described herein below.

The relative amount of SEDS component present within the oral product may vary and is typically selected so as to provide the desired sensory and performance characteristics to the overall oral product. In some embodiments, the oral product comprises up to about 15% by weight of the SEDS, such as from about 1% to about 15%, or from about 1 to about 10% by weight, based on the total weight of the oral product. In some embodiments, the oral product comprises from about 2 to about 5% SEDS by weight, based on the total weight of the oral product.

In some embodiments, at least a portion of the SEDS component is adsorbed or absorbed in or on a component of the overall composition, such as a filler or flow aid as described herein below. In particular embodiments, at least a portion of the SEDS component is adsorbed in or on the flow aid. In some embodiments, the flow aid is silica, and the entirety of the SEDS component is adsorbed in or on the silica.

### Active Ingredient

In some embodiments, the SEDS component, the oral product, or both include one or more active ingredients. In some embodiments, one or more active ingredients is present in the SEDS component.

In some embodiments, one or more active ingredients is present in the composition comprising the oral product. In some embodiments, one or more active ingredients are present in both the SEDS component and the composition comprising the oral product. The active ingredient(s) present in each may be the same or may be different.

As used herein, an "active ingredient" refers to one or more substances belonging to any of the following categories: API (active pharmaceutical substances), food additives, natural medicaments, and naturally occurring substances that can have an effect on humans. Example active ingredients include any ingredient known to impact one or more biological functions within the body, such as ingredients that furnish pharmacological activity or other direct effect in the diagnosis, cure, mitigation, treatment, or prevention of disease, or which affect the structure or any function of the body of humans (e.g., provide a stimulating action on the central nervous system, have an energizing effect, an antipyretic or analgesic action, or an otherwise useful effect on the body). In some embodiments, the active ingredient may be of the type generally referred to as dietary supplements, nutraceuticals, "phytochemicals" or "functional foods". These types of additives are sometimes defined in the art as encompassing substances typically available from naturally occurring sources (e.g., botanical materials) that provide one or more advantageous biological effects (e.g., health promotion, disease prevention, or other medicinal properties), but are not classified or regulated as drugs.

Non-limiting examples of active ingredients include those falling in the categories of botanical ingredients, stimulants, amino acids, nicotine components, and/or pharmaceutical, nutraceutical, and medicinal ingredients (e.g., vitamins, such as B6, B12, and C, and/or cannabinoids, such as tetrahydrocannabinol (THC) and cannabidiol (CBD)). Each of these categories is further described herein below. The particular choice of active ingredients will vary depending upon the desired flavor, texture, and desired characteristics of the particular oral product.

The particular percentages of active ingredients present will vary depending upon the desired characteristics of the particular oral product. Typically, an active ingredient or combination thereof is present in a total concentration of at least about 0.001% by weight of the oral product, such as in a range from about 0.001% to about 20%. In some embodiments, the active ingredient or combination of active ingredients is present in a concentration from about 0.1% w/w to about 10% by weight, such as, e.g., from about from about 0.5% w/w to about 10%, from about 1% to about 10%, from about 1% to about 5% by weight, based on the total weight of the oral product. In some embodiments, the active ingredient or combination of active ingredients is present in a concentration of from about 0.001%, about 0.01%, about 0.1%, or about 1%, up to about 20% by weight, such as, e.g., from about from about 0.001%, about 0.002%, about 0.003%, about 0.004%, about 0.005%, about 0.006%, about 0.007%, about 0.008%, about 0.009%, about 0.01%, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20% by weight, based on the total weight of the oral product. Further suitable ranges for specific active ingredients are provided herein below.

### Nicotine component

In some embodiments, the active ingredient comprises a nicotine component. By "nicotine component" is meant any suitable form of nicotine (e.g., free base or salt) for providing oral absorption of at least a portion of the nicotine present. The source of the nicotine may vary, and may be natural or synthetic. Nicotine may be tobacco-derived (e.g., a tobacco extract) or non-tobacco derived (e.g., synthetic or otherwise obtained). In some embodiments, the nicotine is naturally occurring and obtained as an extract from a *Nicotiana* species (e.g., tobacco). The nicotine can have the enantiomeric form *S*(-)-nicotine, R(+)-nicotine, or a mixture of S(-)-nicotine and R(+)-nicotine. In some embodiments, the nicotine is in the form of S(-)-nicotine (e.g., in a form that is virtually all S(-)-nicotine) or a racemic mixture composed primarily or predominantly of S(-)-nicotine (e.g., a mixture composed of about 95 weight parts S(-)-nicotine and about 5 weight parts R(+)-nicotine). Typically, the nicotine is employed in virtually pure form or in an essentially pure form. In some embodiments, nicotine that is employed has a purity of greater than about 95 percent, such as greater than about 98 percent, or greater than about 99 percent, on a weight basis.

Typically, the nicotine component is selected from the group consisting of nicotine free base and a nicotine salt. In some embodiments, the nicotine component is nicotine in its free base form, which easily can be adsorbed in for example, a microcrystalline cellulose material to form a microcrystalline cellulose-nicotine carrier complex. See, for example, the discussion of nicotine in free base form in US Pat. Pub. No. 2004/0191322 to Hansson, which is incorporated herein by reference.

In some embodiments, at least a portion of the nicotine component can be employed in the form of a salt. Salts of nicotine can be provided using the types of ingredients and techniques set forth in US Pat. No. 2,033,909 to Cox et al., and Perfetti, Beitrage Tabakforschung Int., 12: 43-54 (1983), which are incorporated herein by reference. Additionally, salts of nicotine are available from sources such as Pfaltz and Bauer, Inc. and K&K Laboratories, Division of ICN Biochemicals, Inc. Typically, the nicotine component is selected from the group consisting of nicotine free base, a nicotine salt such as hydrochloride, dihydrochloride, monotartrate, bitartrate, sulfate, salicylate, and nicotine zinc chloride. In some embodiments, the nicotine component is nicotine bitartrate.

In some embodiments, at least a portion of the nicotine can be in the form of a resin complex of nicotine, where nicotine is bound in an ion-exchange resin, such as nicotine polacrilex, which is nicotine bound to, for example, a polymethacrylic acid, such as Amberlite^{™} IRP64, Purolite^{®} C115HMR, or Doshion P551. See, for example, US Pat. No. 3,901,248 to Lichtneckert et al., which is incorporated herein by reference. Another example is a nicotine-polyacrylic carbomer complex, such as with Carbopol^{®} 974P. In some embodiments, nicotine may be present in the form of a nicotine polyacrylic complex.

In some embodiments, at least a portion of the nicotine component can be ion paired as described further herein below.

Typically, the nicotine component (calculated as the free base) when present, is in a concentration of at least about 0.001% by weight of the oral product, such as in a range from about 0.001% to about 10%. In some embodiments, the nicotine component is present in a concentration from about 0.1% w/w to about 10% by weight, such as, e.g., from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, or about 10% by weight, calculated as the free base and based on the total weight of the oral product. In some embodiments, the nicotine component is present in a concentration from about 0.1% w/w to about 3% by weight, such as, e.g., from about 0.1% w/w to about 2.5%, from about 0.1% to about 2.0%, from about 0.1% to about 1.5%, or from about 0.1% to about 1% by weight, calculated as the free base and based on the total weight of the oral product. The total amount of nicotine present may be provided by more than one source of nicotine, such as various combinations of any of nicotine free base, nicotine salt, ion paired nicotine, and polymer-bound nicotine (e.g., nicotine polacrilex). In some embodiments, the nicotine component is nicotine benzoate, nicotine polacrilex, or a mixture thereof.

In some embodiments, the oral product of the disclosure can be characterized as completely free or substantially free of any nicotine component (e.g., any embodiment as disclosed herein may be completely or substantially free of any nicotine component). By "substantially free" is meant that no nicotine has been intentionally added, beyond trace amounts that may be naturally present in e.g., a botanical material. For example, some embodiments can be characterized as having less than 0.001% by weight of nicotine, or less than 0.0001%, or even 0% by weight of nicotine, calculated as the free base.

### Botanical

In some embodiments, the active ingredient comprises a botanical ingredient. As used herein, the term "botanical ingredient" or "botanical" refers to any plant material or fungal-derived material, including plant material in its natural form and plant material derived from natural plant materials, such as extracts or isolates from plant materials or treated plant materials (e.g., plant materials subjected to heat treatment, fermentation, bleaching, or other treatment processes capable of altering the physical and/or chemical nature of the material). For the purposes of the present disclosure, a "botanical" includes, but is not limited to, "herbal materials," which refer to seed-producing plants that do not develop persistent woody tissue and are often valued for their medicinal or sensory characteristics (e.g., teas or tisanes). Reference to botanical material as "non-tobacco" is intended to exclude tobacco materials (i.e., does not include any *Nicotiana* species). The botanical materials used in the present disclosure may comprise, without limitation, any of the compounds and sources set forth herein, including mixtures thereof. Some botanical materials of this type are sometimes referred to as dietary supplements, nutraceuticals, "phytochemicals" or "functional foods."

When present, a botanical is typically at a concentration of from about 0.01% w/w to about 10% by weight, such as, e.g., from about from about 0.01% w/w, about 0.05%, about 0.1%, or about 0.5%, to about 1%, about 2%, about 3%, about 4%, about 5%, about 6%, about 7%, about 8%, about 9%, or about 10%, about 11%, about 12%, about 13%, about 14%, or about 15% by weight, based on the total weight of the oral product.

The botanical materials useful in the present disclosure may comprise, without limitation, any of the compounds and sources set forth herein, including mixtures thereof. Certain botanical materials of this type are sometimes referred to as dietary supplements, nutraceuticals, "phytochemicals" or "functional foods." Certain botanicals, as the plant material or an extract thereof, have found use in traditional herbal medicine, and are described further herein.

Non-limiting examples of non-tobacco botanical materials include without limitation acai berry (Euterpe oleracea martius), acerola (Malpighia glabra), alfalfa, allspice, Angelica root, anise (e.g., star anise), annatto seed, apple (*Malus domestica*)*,* apricot oil, ashwagandha, *Bacopa monniera*, baobab, basil (*Ocimum basilicum*), bay, bee balm, beet root, bergamot, blackberry (*Morus nigra*), black cohosh, black pepper, black tea, blueberries, boldo (*Peumus boldus*), borage, bugleweed, cacao, calamus root, camu (*Myrcaria dubia*), cannabis/hemp, caraway seed, cardamom, cassis, catnip, catuaba, cayenne pepper, *Centella asiatica,* chaga mushroom, Chai-hu, chamomile, cherry, chervil, chive, chlorophyll, chocolate, cilantro, cinnamon (*Cinnamomum cassia*), citron grass (*Cymbopogon citratus*), citrus, clary sage, cloves, coconut (*Cocos nucifera*), coffee, comfrey leaf and root, cordyceps, coriander seed, cranberry, cumin, curcumin, damiana, dandelion, *Dorstenia arifolia*, *Dorstenia odorata*, Echinacea, elderberry, elderflower, endro (*Anethum graveolens*), evening primrose, eucalyptus, fennel, feverfew, flax, *Galphimia glauca*, garlic, ginger (*Zingiber officinale*), gingko biloba, ginseng, goji berries, goldenseal, grape seed, grapefruit, grapefruit rosé (*Citrus paradisi*), graviola (*Annona muricata*), green tea, guarana, gutu kola, hawthorn, hazel, hemp, hibiscus flower (*Hibiscus sabdariffa*), honeybush, hops, jiaogulan, jambu (*Spilanthes oleraceae*)*,* jasmine (*Jasminum officinale*), juniper berry (*Juniperus communis*)*, Kaempferia parviflora* (Thai ginseng), kava, laurel, lavender, lemon (*Citrus limon*), lemon balm, lemongrass, licorice, lilac, Lion's mane, lutein, maca (*Lepidium meyenii*), mace, marjoram, matcha, milk thistle, mints (menthe), mulberry, *Nardostachys chinensis,* nutmeg, olive, oolong tea, orange (*Citrus sinensis*), oregano, papaya, paprika, pennyroyal, peppermint (*Mentha piperita*), pimento, potato peel, primrose, quercetin, quince, red clover, resveratrol, *Rhizoma gastrodiae*, *Rhodiola*, rooibos (red or green), rosehip (*Rosa canina*), rosemary, saffron, sage, Saint John's Wort, sandalwood, salvia (*Salvia officinalis*), savory, saw palmetto, *Sceletium tortuosum,* Schisandra, silybum marianum, Skullcap, spearmint, Spikenard, spirulina, slippery elm bark, sorghum bran hi-tannin, sorghum grain hi-tannin, spearmint (*Mentha spicata*), spirulina, star anise, sumac bran, tarragon, thyme, tisanes, turmeric, *Turnera aphrodisiaca*, uva ursi, valerian, vanilla, *Viola odorata*, wild yam root, wintergreen, withania somnifera, yacon root, yellow dock, yerba mate, and yerba santa.

In some embodiments, the oral product of the disclosure includes a tobacco material (such as a whitened tobacco material). In some embodiments, the oral product can be characterized as completely free or substantially free of any tobacco material (exclusive of nicotine, if present). By "substantially free" is meant that no tobacco material has been intentionally added. For example, some embodiments can be characterized as having less than 0.001% by weight of tobacco material, or less than 0.0001%, or even 0% by weight of tobacco material, calculated as the free base.

### Stimulants

In some embodiments, the active ingredient comprises one or more stimulants. As used herein, the term "stimulant" refers to a material that increases activity of the central nervous system and/or the body, for example, enhancing focus, cognition, vigor, mood, alertness, and the like. Non-limiting examples of stimulants include caffeine, theacrine, theobromine, and theophylline. Theacrine (1,3,7,9-tetramethyluric acid) is a purine alkaloid which is structurally related to caffeine, and possesses stimulant, analgesic, and anti-inflammatory effects. Present stimulants may be natural, naturally derived, or wholly synthetic. For example, certain botanical materials (guarana, tea, coffee, cocoa, and the like) may possess a stimulant effect by virtue of the presence of e.g., caffeine or related alkaloids, and accordingly are "natural" stimulants. By "naturally derived" is meant the stimulant (e.g., caffeine, theacrine) is in a purified form, outside its natural (e.g., botanical) matrix. For example, caffeine can be obtained by extraction and purification from botanical sources (e.g., tea). By "wholly synthetic", it is meant that the stimulant has been obtained by chemical synthesis. In some embodiments, the active ingredient comprises caffeine. In some embodiments, the active ingredient is caffeine. In some embodiments, the caffeine is present in an encapsulated form. On example of an encapsulated caffeine is Vitashure^{®}, available from Balchem Corp., 52 Sunrise Park Road, New Hampton, NY, 10958.

When present, a stimulant or combination of stimulants (e.g., caffeine, theacrine, and combinations thereof) is typically at a concentration of from about 0.1% w/w to about 15% by weight, such as, e.g., from about from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 11%, about 12%, about 13%, about 14%, or about 15% by weight, based on the total weight of the oral product.

### Amino acids

In some embodiments, the active ingredient comprises an amino acid. As used herein, the term "amino acid" refers to an organic compound that contains amine (-NH₂) and carboxyl (-COOH) or sulfonic acid (SO₃H) functional groups, along with a side chain (R group), which is specific to each amino acid. Amino acids may be proteinogenic or non-proteinogenic. By "proteinogenic" is meant that the amino acid is one of the twenty naturally occurring amino acids found in proteins. The proteinogenic amino acids include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine. By "non-proteinogenic" is meant that either the amino acid is not found naturally in protein, or is not directly produced by cellular machinery (e.g., is the product of post-translational modification). Non-limiting examples of non-proteinogenic amino acids include gamma-aminobutyric acid (GABA), taurine (2-aminoethanesulfonic acid), theanine (L-γ-glutamylethylamide), hydroxyproline, and beta-alanine.

When present, an amino acid or combination of amino acids (e.g., taurine, theanine, and combinations thereof) is typically at a concentration of from about 0.1% w/w to about 15% by weight, such as, e.g., from about from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 11%, about 12%, about 13%, about 14%, or about 15% by weight, based on the total weight of the oral product.

### Vitamins and Minerals

In some embodiments, the active ingredient comprises a vitamin or combination of vitamins. As used herein, the term "vitamin" refers to an organic molecule (or related set of molecules) that is an essential micronutrient needed for the proper functioning of metabolism in a mammal. There are thirteen vitamins required by human metabolism, which are: vitamin A (as all-trans-retinol, all-trans-retinyl-esters, as well as all-trans-beta-carotene and other provitamin A carotenoids), vitamin B1 (thiamine), vitamin B2 (riboflavin), vitamin B3 (niacin), vitamin B5 (pantothenic acid), vitamin B6 (pyridoxine), vitamin B7 (biotin), vitamin B9 (folic acid or folate), vitamin B12 (cobalamins), vitamin C (ascorbic acid), vitamin D (calciferols), vitamin E (tocopherols and tocotrienols), and vitamin K (quinones).

When present, a vitamin or combination of vitamins (e.g., vitamin B6, vitamin B12, vitamin E, vitamin C, or a combination thereof) is typically at a concentration of from about 0.01% w/w to about 1% by weight, such as, e.g., from about 0.01%, about 0.02%, about 0.03%, about 0.04%, about 0.05%, about 0.06%, about 0.07%, about 0.08%, about 0.09%, or about 0.1% w/w, to about 0.2%, about 0.3%, about 0.4%, about 0.5% about 0.6%, about 0.7%, about 0.8%, about 0.9%, or about 1% by weight, based on the total weight of the oral product.

In some embodiments, the active ingredient comprises a mineral. As used herein, the term "mineral" refers to an inorganic molecule (or related set of molecules) that is an essential micronutrient needed for the proper functioning of various systems in a mammal. Non-limiting examples of minerals include iron, zinc, copper, selenium, chromium, cobalt, manganese, calcium, phosphorus, sulfur, magnesium, and the like. In some embodiments, the active ingredient comprises iron. Suitable sources of iron include, but are not limited to, ferrous salts such as ferrous sulfate and ferrous gluconate. In some embodiments, the iron is encapsulated.

### Cannabinoids

In some embodiments, the active ingredient comprises one or more cannabinoids. As used herein, the term "cannabinoid" refers to a class of diverse natural or synthetic chemical compounds that acts on cannabinoid receptors (i.e., CB1 and CB2) in cells that alter neurotransmitter release in the brain. Cannabinoids are cyclic molecules exhibiting particular properties such as the ability to easily cross the blood-brain barrier. Cannabinoids may be naturally occurring (Phytocannabinoids) from plants such as cannabis, (endocannabinoids) from animals, or artificially manufactured (synthetic cannabinoids). Cannabis species express at least 85 different phytocannabinoids, and these may be divided into subclasses, including cannabigerols, cannabichromenes, cannabidiols, tetrahydrocannabinols, cannabinols and cannabinodiols, and other cannabinoids, such as cannabigerol (CBG), cannabichromene (CBC), cannabidiol (CBD), tetrahydrocannabinol (THC), cannabinol (CBN) and cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM), cannabinerolic acid, cannabidiolic acid (CBDA), cannabinol propyl variant (CBNV), cannabitriol (CBO), tetrahydrocannabmolic acid (THCA), and tetrahydrocannabivarinic acid (THCV A).

In some embodiments, the cannabinoid is selected from the group consisting of cannabigerol (CBG), cannabichromene (CBC), cannabidiol (CBD), tetrahydrocannabinol (THC), cannabinol (CBN) and cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM), cannabinerolic acid, cannabidiolic acid (CBDA), Cannabinol propyl variant (CBNV), cannabitriol (CBO), tetrahydrocannabinolic acid (THCA), tetrahydrocannabivarinic acid (THCV A), and mixtures thereof. In some embodiments, the cannabinoid comprises at least tetrahydrocannabinol (THC). In some embodiments, the cannabinoid is tetrahydrocannabinol (THC). In some embodiments, the cannabinoid comprises at least cannabidiol (CBD). In some embodiments, the cannabinoid is cannabidiol (CBD). In some embodiments, the CBD is synthetic CBD.

In some embodiments, the cannabinoid (e.g., CBD) is added to the composition in the form of an isolate. An isolate is an extract from a plant, such as cannabis, where the active material of interest (in this case the cannabinoid, such as CBD) is present in a high degree of purity, for example greater than 95%, greater than 96%, greater than 97%, greater than 98%, or around 99% purity.

In some embodiments, the cannabinoid is an isolate of CBD in a high degree of purity, and the amount of any other cannabinoid in the oral product is no greater than about 1% by weight of the oral product, such as no greater than about 0.5% by weight of the oral product, such as no greater than about 0.1% by weight of the oral product, such as no greater than about 0.01% by weight of the oral product.

The choice of cannabinoid and the particular percentages thereof which may be present within the disclosed oral product will vary depending upon the desired flavor, texture, and other characteristics of the oral product.

In some embodiments, the cannabinoid (such as CBD) is present in the oral product in a concentration of at least about 0.001% by weight of the oral product, such as in a range from about 0.001% to about 2% by weight of the oral product. In some embodiments, the cannabinoid (such as CBD) is present in the oral product in a concentration of from about 0.1% to about 1.5% by weight, based on the total weight of the oral product. In some embodiments, the cannabinoid (such as CBD) is present in a concentration from about 0.4% to about 1.5% by weight, based on the total weight of the oral product.

Alternatively, or in addition to the cannabinoid, the active ingredient may include a cannabimimetic, which is a class of compounds derived from plants other than cannabis that have biological effects on the endocannabinoid system similar to cannabinoids. Examples include yangonin, alpha-amyrin or beta-amyrin (also classified as terpenes), cyanidin, curcumin (tumeric), catechin, quercetin, salvinorin A, N-acylethanolamines, and N-alkylamide lipids. Such compounds can be used in the same amounts and ratios noted herein for cannabinoids.

### Terpenes

Active ingredients suitable for use in the present disclosure can also be classified as terpenes, many of which are associated with biological effects, such as calming effects. Terpenes are understood to have the general formula of (C₅H₈)ₙ and include monoterpenes, sesquiterpenes, and diterpenes. Terpenes can be acyclic, monocyclic or bicyclic in structure. Some terpenes provide an entourage effect when used in combination with cannabinoids or cannabimimetics. Examples include beta-caryophyllene, linalool, limonene, beta-citronellol, linalyl acetate, pinene (alpha or beta), geraniol, carvone, eucalyptol, menthone, iso-menthone, piperitone, myrcene, beta-bourbonene, and germacrene, which may be used singly or in combination.

In some embodiments, the terpene is a terpene derivable from a phytocannabinoid producing plant, such as a plant from the stain of the cannabis sativa species, such as hemp. Suitable terpenes in this regard include so-called "C10" terpenes, which are those terpenes comprising 10 carbon atoms, and so-called "C15" terpenes, which are those terpenes comprising 15 carbon atoms. In some embodiments, the active ingredient comprises more than one terpene. For example, the active ingredient may comprise one, two, three, four, five, six, seven, eight, nine, ten or more terpenes as defined herein. In some embodiments, the terpene is selected from pinene (alpha and beta), geraniol, linalool, limonene, carvone, eucalyptol, menthone, iso-menthone, piperitone, myrcene, beta-bourbonene, germacrene and mixtures thereof.

### Pharmaceutical ingredients

In some embodiments, the active ingredient comprises an active pharmaceutical ingredient (API). The API can be any known agent adapted for therapeutic, prophylactic, or diagnostic use. These can include, for example, synthetic organic compounds, proteins and peptides, polysaccharides and other sugars, lipids, phospholipids, inorganic compounds (e.g., magnesium, selenium, zinc, nitrate), neurotransmitters or precursors thereof (e.g., serotonin, 5-hydroxytryptophan, oxitriptan, acetylcholine, dopamine, melatonin), and nucleic acid sequences, having therapeutic, prophylactic, or diagnostic activity. Non-limiting examples of APIs include analgesics and antipyretics (e.g., acetylsalicylic acid, acetaminophen, 3-(4-isobutylphenyl)propanoic acid), phosphatidylserine, myoinositol, docosahexaenoic acid (DHA, Omega-3), arachidonic acid (AA, Omega-6), S-adenosylmethionine (SAM), beta-hydroxy-beta-methylbutyrate (HMB), citicoline (cytidine-5'-diphosphate-choline), and cotinine.

The amount of API may vary. For example, when present, an API is typically at a concentration of from about 0.001% w/w to about 10% by weight, such as, e.g., from about from about 0.01%, about 0.02%, about 0.03%, about 0.04%, about 0.05%, about 0.06%, about 0.07%, about 0.08%, about 0.09%, about 0.1% w/w, about 0.2%, about 0.3%, about 0.4%, about 0.5% about 0.6%, about 0.7%, about 0.8%, about 0.9%, or about 1%, to about 2%, about 3%, about 4%, about 5%, about 6%, about 7%, about 8%, about 9%, or about 10% by weight, based on the total weight of the oral product.

### Ion Pairing of Basic Amine-Containing Active Ingredients and Organic Acids

In some embodiments, the active ingredient has a basic amine functionality (i.e., the active ingredient comprises a basic amine). By "basic amine" is meant a molecule including at least one basic amine functional group. Examples of basic amines include, but are not limited to, alkaloids. By "basic amine functional group" is meant a group containing a nitrogen atom having a lone pair of electrons. The basic amine functional group is attached to or incorporated within the molecule through one or more covalent bonds to the said nitrogen atom. The basic amine may be a primary, secondary, or tertiary amine, meaning the nitrogen bears one, two, or three covalent bonds to carbon atoms. By virtue of the lone pair of electrons on the nitrogen atom, such amines are termed "basic", meaning the lone electron pair is available for hydrogen bonding. The basicity (i.e., the electron density on the nitrogen atom and consequently the availability and strength of hydrogen bonding to the nitrogen atom) of the basic amine may be influenced by the nature of neighboring atoms, the steric bulk of the molecule, and the like.

Generally, the basic amine is released from the composition and absorbed through the oral mucosa, thereby entering the blood stream, where it is circulated systemically. Generally, the basic amine is present in or as an active ingredient in the composition, as described herein below.

In some embodiments, the active ingredient having the basic amine functionality is caffeine. In some embodiments, the active ingredient having a basic amine functionality is nicotine, for example, a nicotine component as described herein above.

In some embodiments, at least a portion the active ingredient having the basic amine functionality (e.g., nicotine) is associated with at least a portion of the organic acid or the alkali metal salt thereof which may be present in some embodiments. Depending on multiple variables (concentration, pH, nature of the organic acid, and the like), the active ingredient having the basic amine functionality present in the oral product can exist in multiple forms, including ion paired, in solution (i.e., fully solvated), as the free base, as a cation, as a salt, or any combination thereof.

In some embodiments, at least a portion of the active ingredient present in the oral product and having the basic amine functionality is in an ion paired form with an organic acid or conjugate base thereof as further described herein below. In some embodiments, the active ingredient having the basic amine functionality and at least a portion of the organic acid or the alkali metal salt thereof is in the form of an ion pair between the basic amine and a conjugate base of the organic acid.

Ion pairing describes the partial association of oppositely charged ions in relatively concentrated solutions to form distinct chemical species called ion pairs. The strength of the association (i.e., the ion pairing) depends on the electrostatic force of attraction between the positive and negative ions (i.e., a protonated basic amine and the conjugate base of an organic acid). By "conjugate base" is meant the base resulting from deprotonation of the corresponding acid (e.g., benzoate is the conjugate base of benzoic acid).

On average, a certain population of these ion pairs exists at any given time, although the formation and dissociation of ion pairs is continuous. In the composition as disclosed herein, and/or upon oral use of said composition (e.g., upon contact with saliva), the active ingredient having the basic amine functionality and the conjugate base of the organic acid exist at least partially in the form of an ion pair. Without wishing to be bound by theory, it is believed that such ion pairing may minimize chemical degradation of the active ingredient having the basic amine functionality and/or enhance the oral availability of the active ingredient having the basic amine functionality. At alkaline pH values (e.g., such as from about 7.5 to about 9), certain active ingredient having the basic amine functionality, for example nicotine, are largely present in the free base form, which has relatively low water solubility, and low stability with respect to evaporation and oxidative decomposition, but high mucosal availability. Conversely, at acidic pH values (such as from about 6.5 to about 4), certain active ingredient having the basic amine functionality, for example nicotine, are largely present in a protonated form, which has relatively high solubility in water, and higher stability with respect to evaporation and oxidative decomposition, but low mucosal availability. In some embodiments, the properties of stability, solubility, and availability of nicotine can be enhanced through ion pairing or salt formation of nicotine with appropriate organic acids and/or their conjugate bases. Specifically, nicotine-organic acid ion pairs of moderate lipophilicity result in favorable stability and absorption properties. Lipophilicity is conveniently measured in terms of logP, the partition coefficient of a molecule between a lipophilic phase and an aqueous phase, usually octanol and water, respectively. An octanol-water partitioning favoring distribution of an ion pair into octanol is predictive of good absorption of the active ingredient having the basic amine functionality present in the composition through the oral mucosa.

As noted above, at alkaline pH values (e.g., such as from about 7.5 to about 9), nicotine is largely present in the free base form (and accordingly, a high partitioning into octanol), while, at acidic pH values (such as from about 6.5 to about 4), nicotine is largely present in a protonated form (and accordingly, a low partitioning into octanol). Surprisingly, according to the present disclosure, it has been found that an ion pair between certain organic acids (e.g., having a logP value of from about 1.4 to about 8.0. such as from about 1.4 to about 4.5, allows nicotine partitioning into octanol consistent with that predicted for nicotine partitioning into octanol at a pH of 8.4.

One of skill in the art will recognize that the extent of ion pairing in the disclosed composition, both before and during use by the consumer, may vary based on, for example, pH, the nature of the organic acid, the concentration of nicotine, the concentration of the organic acid or conjugate base of the organic acid present in the composition, the moisture content of the composition, the ionic strength of the composition, and the like. One of skill in the art will also recognize that ion pairing is an equilibrium process influenced by the foregoing variables. Accordingly, quantification of the extent of ion pairing is difficult or impossible by calculation or direct observation. However, the presence of ion pairing may be demonstrated through surrogate measures such as partitioning between octanol and water or membrane permeation of aqueous solutions of nicotine plus organic acids and/or their conjugate bases.

### Organic acid

As discussed herein above, in some embodiments, the oral product as disclosed herein comprises an organic acid, an alkali metal salt thereof, or both. In some embodiments, at least a portion of the organic acid or salt thereof is in the form of an ion pair with a basic amine-containing active ingredient as described herein above.

As used herein, the term "organic acid" refers to an organic (i.e., carbon-based) compound that is characterized by acidic properties. Typically, organic acids are relatively weak acids (i.e., they do not dissociate completely in the presence of water), such as carboxylic acids (-CO₂H) or sulfonic acids (-SO₂OH). As used herein, reference to organic acid means an organic acid that is intentionally added. In this regard, an organic acid may be intentionally added as a specific composition ingredient as opposed to merely being inherently present as a component of another composition ingredient (e.g., the small amount of organic acid which may inherently be present in a composition ingredient, such as a tobacco material).

Suitable organic acids will typically have a range of lipophilicities (i.e., a polarity giving an appropriate balance of water and organic solubility). Typically, lipophilicities of suitable organic acids, as indicated by logP, will vary between about 1 and about 12 (more soluble in octanol than in water). In some embodiments, the organic acid has a logP value from about 1 to about 12, e.g., from about 1.0. about 1.5, about 2.0, about 2.5, about 3.0, about 3.5, about 4.0, about 4.5, about 5.0, about 5.5, about 6.0, about 6.5, about 7.0, about 7.5, or about 8.0, to about 8.5, about 9.0, about 9.5, about 10.0, about 10.5, about 11.0, about 11.5, or about 12.0.

Without wishing to be bound by theory, it is believed that moderately lipophilic organic acids (e.g., logP of from about 1.4 to about 4.5) produce ion pairs with nicotine which are of a polarity providing good octanol-water partitioning of the ion pair, and hence partitioning of nicotine, into octanol versus water. As discussed above, such partitioning into octanol is predictive of favorable oral availability.

In some embodiments, the organic acid has a logP value from about 3.0 to about 8.0, about 10.0, or even 12.0. In some embodiments, the presence of certain solvents or solubilizing agents (e.g., inclusion in the oral product of glycerin or propylene glycol) may be beneficial in solubilizing organic acids and the corresponding salts or ion pairs thereof with the basic amine for highly lipophilic organic acids (e.g., higher than about 4.5).

In some embodiments, the organic acid is a carboxylic acid or a sulfonic acid. The carboxylic acid or sulfonic acid functional group may be attached to any alkyl, cycloalkyl, heterocycloalkyl, aryl, or heteroaryl group having, for example, from one to twenty carbon atoms (C1-C20). In some embodiments, the organic acid is an alkyl, cycloalkyl, heterocycloalkyl, aryl, or heteroaryl carboxylic or sulfonic acid.

As used herein, "alkyl" refers to any straight chain or branched chain hydrocarbon. The alkyl group may be saturated (i.e., having all sp3 carbon atoms), or may be unsaturated (i.e., having at least one site of unsaturation). As used herein, the term "unsaturated" refers to the presence of a carbon-carbon, sp2 double bond in one or more positions within the alkyl group. Unsaturated alkyl groups may be mono- or polyunsaturated. Representative straight chain alkyl groups include, but are not limited to, methyl, ethyl, n-propyl, n-butyl, n-pentyl, and n-hexyl. Branched chain alkyl groups include, but are not limited to, isopropyl, sec-butyl, isobutyl, tert-butyl, isopentyl, and 2-methylbutyl. Representative unsaturated alkyl groups include, but are not limited to, ethylene or vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like. An alkyl group can be unsubstituted or substituted.

"Cycloalkyl" as used herein refers to a carbocyclic group, which may be mono- or bicyclic. Cycloalkyl groups include rings having 3 to 7 carbon atoms as a monocycle or 7 to 12 carbon atoms as a bicycle. Examples of monocyclic cycloalkyl groups include cyclopropyl, cyclobutyl, cyclopentyl, cyclohexyl, cycloheptyl, and cyclooctyl. A cycloalkyl group can be unsubstituted or substituted, and may include one or more sites of unsaturation (e.g., cyclopentenyl or cyclohexenyl).

The term "aryl" as used herein refers to a carbocyclic aromatic group. Examples of aryl groups include, but are not limited to, phenyl and naphthyl. An aryl group can be unsubstituted or substituted.

"Heteroaryl" and "heterocycloalkyl" as used herein refer to an aromatic or non-aromatic ring system, respectively, in which one or more ring atoms is a heteroatom, e.g. nitrogen, oxygen, and sulfur. The heteroaryl or heterocycloalkyl group comprises up to 20 carbon atoms and from 1 to 3 heteroatoms selected from N, O, and S. A heteroaryl or heterocycloalkyl may be a monocycle having 3 to 7 ring members (for example, 2 to 6 carbon atoms and 1 to 3 heteroatoms selected from N, O, and S) or a bicycle having 7 to 10 ring members (for example, 4 to 9 carbon atoms and 1 to 3 heteroatoms selected from N, O, and S), for example: a bicyclo[4,5], [5,5], [5,6], or [6,6] system. Examples of heteroaryl groups include by way of example and not limitation, pyridyl, thiazolyl, tetrahydrothiophenyl, pyrimidinyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, isoxazolyl, pyrazinyl, pyridazinyl, indolizinyl, isoindolyl, 3H-indolyl, 1H-indazolyl, purinyl, 4H-quinolizinyl, phthalazinyl, naphthyridinyl, quinoxalinyl, quinazolinyl, cinnolinyl, pteridinyl, 4aH-carbazolyl, carbazolyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, benzotriazolyl, benzisoxazolyl, and isatinoyl. Examples of heterocycloalkyls include by way of example and not limitation, dihydroypyridyl, tetrahydropyridyl (piperidyl), tetrahydrothiophenyl, piperidinyl, 4-piperidonyl, pyrrolidinyl, 2-pyrrolidonyl, tetrahydrofuranyl, tetrahydropyranyl, bis-tetrahydropyranyl, tetrahydroquinolinyl, tetrahydroisoquinolinyl, decahydroquinolinyl, octahydroisoquinolinyl, piperazinyl, quinuclidinyl, and morpholinyl. Heteroaryl and heterocycloalkyl groups can be unsubstituted or substituted.

"Substituted" as used herein and as applied to any of the above alkyl, aryl, cycloalkyl, heteroaryl, heterocyclyl, means that one or more hydrogen atoms are each independently replaced with a substituent. Typical substituents include, but are not limited to, -Cl, Br, F, alkyl, -OH, -OCH₃, NH₂, -NHCH₃,-N(CH₃)₂, -CN, -NC(=O)CH₃, -C(=O)-, -C(=O)NH₂, and -C(=O)N(CH₃)₂. Wherever a group is described as "optionally substituted," that group can be substituted with one or more of the above substituents, independently selected for each occasion. In some embodiments, the substituent may be one or more methyl groups or one or more hydroxyl groups.

In some embodiments, the organic acid is an alkyl carboxylic acid. Non-limiting examples of alkyl carboxylic acids include formic acid, acetic acid, propionic acid, butyric acid, valeric acid, caproic acid, heptanoic acid, octanoic acid, nonanoic acid, decanoic acid, undecanoic acid, dodecanoic acid, stearic acid, oleic acid, linoleic acid, linolenic acid, and the like.

In some embodiments, the organic acid is an alkyl sulfonic acid. Non-limiting examples of alkyl sulfonic acids include propanesulfonic acid, heptanesulfonic acid, and octanesulfonic acid.

In some embodiments, the alkyl carboxylic or sulfonic acid is substituted with one or more hydroxyl groups. Non-limiting examples include glycolic acid, 4-hydroxybutyric acid, and lactic acid.

In some embodiments, an organic acid may include more than one carboxylic acid group or more than one sulfonic acid group (e.g., two, three, or more carboxylic acid groups). Non-limiting examples include oxalic acid, fumaric acid, maleic acid, and glutaric acid. In organic acids containing multiple carboxylic acids (e.g., from two to four carboxylic acid groups), one or more of the carboxylic acid groups may be esterified. Non-limiting examples include succinic acid monoethyl ester, monomethyl fumarate, monomethyl or dimethyl citrate, and the like.

In some embodiments, the organic acid may include more than one carboxylic acid group and one or more hydroxyl groups. Non-limiting examples of such acids include tartaric acid, citric acid, and the like.

In some embodiments, the organic acid is an aryl carboxylic acid or an aryl sulfonic acid. Non-limiting examples of aryl carboxylic and sulfonic acids include benzoic acid, toluic acids, salicylic acid, benzenesulfonic acid, and p-toluenesulfonic acid.

Further non-limiting examples of organic acids which may be useful in some embodiments include dibenzoyl-L-tartaric acid, 2,2-dichloroacetic acid, 2-hydroxyethanesulfonic acid, 2-oxoglutaric acid, 4-acetamidobenzoic acid, 4-aminosalicylic acid, adipic acid, ascorbic acid (L), aspartic acid (L), alpha-methylbutyric acid, camphoric acid (+), camphor-10-sulfonic acid (+), cinnamic acid, cyclamic acid, dodecylsulfuric acid, ethane-1,2-disulfonic acid, ethanesulfonic acid, furoic acid, galactaric acid, gentisic acid, glucoheptonic acid, gluconic acid, glucuronic acid, glutamic acid, glycerophosphoric acid, glycolic acid, hippuric acid, isobutyric acid, isovaleric acid, lactobionic acid, lauric acid, levulinic acid, malic acid, malonic acid, mandelic acid, methanesulfonic acid, naphthalene-1,5-disulfonic acid, naphthalene-2-sulfonic acid, oleic acid, palmitic acid, pamoic acid, phenylacetic acid, pyroglutamic acid, pyruvic acid, sebacic acid, stearic acid, and undecylenic acid. Examples of suitable acids include, but are not limited to, the list of organic acids in **Table 1**.

**Table 1. Non-limiting examples of suitable organic acids**

| **Acid Name** | **log(P)*** |
|---|---|
| benzoic acid | 1.9 |
| phenylacetic | 1.4 |
| p-toluic acid | 2.3 |
| ethyl benzoic acid | 2.9 |
| isopropyl benzoic acid | 3.5 |
| 4-phenylbutyric | 2.4 |
| 2-(4-Isobutylphenyl)propanoic acid | 3.5 |
| 2-napthoxyacetic acid | 2.5 |
| napthylacetic acid | 2.7 |
| heptanoic acid | 2.5 |
| octanoic acid | 3.05 |
| nonanoic acid | 3.5 |
| decanoic acid | 4.09 |
| 9-deceneoic acid | 3.3 |
| 2-deceneoic acid | 3.8 |
| 10-undecenoic acid | 3.9 |
| dodecandioic acid | 3.2 |
| dodecanoic acid | 4.6 |
| myristic acid | 5.3 |
| palmitic acid | 6.4 |
| stearic acid | 7.6 |
| cyclohexanebutanoic acid | 3.4 |
| 1-heptanesulfonic acid | 2.0 |
| 1-octanesulfonic acid | 2.5 |
| 1-nonanesulfonic acid | 3.1 |
| monooctyl succinate | 2.8 |
| tocopherol succinate | 10.2 |
| monomenthyl succinate | 3 |
| monomenthyl glutarate | 3.4 |
| norbixin ((2*E*,4*E*,6*E*,8*E*,10*E*,12*E*,14*E*,16*E*,18*E*)-4,8,13,17-tetramethylicosa-2,4,6,8,10,12,14,16,18-nonaenedioic acid) | 7.2 |
| bixin ((2E,4E,6E,8E,10E,12E,14E,16Z,18E)-20-methoxy-4,8,13,17-tetramethyl-20-oxoicosa-2,4,6,8,10,12,14,16,18-nonaenoic acid) | 7.5 |

| | |
|---|---|
| *Values obtained from PubChem or calculated | |

The selection of organic acid may further depend on additional properties in addition to consideration of the logP value. For example, an organic acid should be one recognized as safe for human consumption, and which has acceptable flavor, odor, volatility, stability, and the like. Determination of appropriate organic acids is within the purview of one of skill in the art.

In some embodiments, the organic acid is a mono ester of a dicarboxylic acid or a poly-carboxylic acid. In some embodiments, the dicarboxylic acid is malonic acid, succinic acid, glutaric acid, adipic acid, fumaric acid, maleic acid, or a combination thereof. In some embodiments, the dicarboxylic acid is succinic acid, glutaric acid, fumaric acid, maleic acid, or a combination thereof. In some embodiments, the dicarboxylic acid is succinic acid, glutaric acid, or a combination thereof.

In some embodiments, the alcohol forming the mono ester of the dicarboxylic acid is a lipophilic alcohol. Examples of suitable lipophilic alcohols include, but are not limited to, octanol, menthol, and tocopherol. In some embodiments, the organic acid is an octyl mono ester of a dicarboxylic acid, such as monooctyl succinate, monooctyl fumarate, or the like. In some embodiments, the organic acid is a monomenthyl ester of a dicarboxylic acid. Certain menthyl esters may be desirable in oral compositions as described herein by virtue of the cooling sensation they may provide upon use of the product comprising the composition. In some embodiments, the organic acid is monomenthyl succinate, monomenthyl fumarate, monomenthyl glutarate, or a combination thereof. In some embodiments, the organic acid is a monotocopheryl ester of a dicarboxylic acid. Certain tocopheryl esters may be desirable in oral compositions as described herein by virtue of the antioxidant effects they may provide. In some embodiments, the organic acid is tocopheryl succinate, tocopheryl fumarate, tocopheryl glutarate, or a combination thereof.

In some embodiments, the organic acid is a carotenoid derivative having one or more carboxylic acids. Carotenoids are tetraterpenes, meaning that they are produced from 8 isoprene molecules and contain 40 carbon atoms. Accordingly, they are usually lipophilic due to the presence of long unsaturated aliphatic chains, and are generally yellow, orange, or red in color. Certain carotenoid derivatives can be advantageous in oral compositions by virtue of providing both ion pairing and serving as a colorant in the composition. In some embodiments, the organic acid is 2*E*,4*E*,6*E*,8*E*,10*E*,12*E*,14*E*,16*Z*,18*E*)-20-methoxy-4,8,13,17-tetramethyl-20-oxoicosa-2,4,6,8,10,12,14,16,18-nonaenoic acid (bixin) or an isomer thereof. Bixin is an apocarotenoid found in annatto seeds from the achiote tree (*Bixa orellana*) and is the naturally occurring pigment providing the reddish orange color to annatto. Bixin is soluble in fats and alcohols but insoluble in water, and is chemically unstable when isolated, converting via isomerization into the double bond isomer, *trans*-bixin (β-bixin), having the structure:

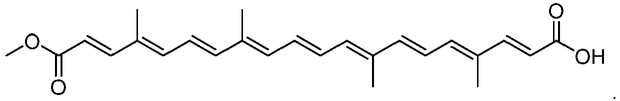

In some embodiments, the organic acid is (2£,4£,6£,8£,10£,12£,14£,16£,18£)-4,8,13,17-tetramethylicosa-2,4,6,8,10,12,14,16,18-nonaenedioic acid (norbixin), a water-soluble hydrolysis product of bixin having the structure:

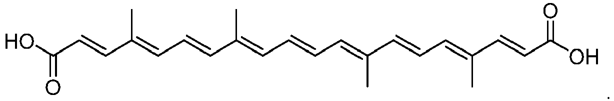

The selection of organic acid may further depend on additional properties in addition to or without consideration to the logP value. For example, an organic acid should be one recognized as safe for human consumption, and which has acceptable flavor, odor, volatility, stability, and the like. Determination of appropriate organic acids is within the purview of one of skill in the art.

In some embodiments, more than one organic acid may be present. For example, the composition may comprise two, or three, or four, or more organic acids. Accordingly, reference herein to "an organic acid" contemplates mixtures of two or more organic acids. The relative amounts of the multiple organic acids may vary. For example, a composition may comprise equal amounts of two, or three, or more organic acids, or may comprise different relative amounts. In this manner, it is possible to include certain organic acids (e.g., citric acid or myristic acid) which have a logP value outside the desired range, when combined with other organic acids to provide the desired average logP range for the combination. In some embodiments, it may be desirable to include organic acids in the composition which have logP values outside the desired range for purposes such as, but not limited to, providing desirable organoleptic properties, stability, as flavor components, and the like. Further, certain lipophilic organic acids have undesirable flavor and or aroma characteristics which would preclude their presence as the sole organic acid (e.g., in equimolar or greater quantities relative to nicotine). Without wishing to be bound by theory, it is believed that a combination of different organic acids may provide the desired ion pairing while the concentration of any single organic acid in the composition remains below the threshold which would be found objectionable from a sensory perspective.

For example, in some embodiments, the organic acid may comprise from about 1 to about 5 or more molar equivalents of benzoic acid relative to nicotine, combined with e.g., about 0.2 molar equivalents of octanoic acid or a salt thereof, and 0.2 molar equivalents of decanoic acid or a salt thereof.

In some embodiments, the organic acid is a combination of any two organic acids selected from the group consisting of benzoic acid, a toluic acid, benzenesulfonic acid, toluenesulfonic acid, hexanoic acid, heptanoic acid, decanoic acid, and octanoic acid. In some embodiments, the organic acid is a combination of benzoic acid, octanoic acid, and decanoic acid, or benzoic and octanoic acid. In some embodiments, the composition comprises citric acid in addition to one or more of benzoic acid, a toluic acid, benzenesulfonic acid, toluenesulfonic acid, hexanoic acid, heptanoic acid, decanoic acid, and octanoic acid.

In some embodiments, the oral product comprises an alkali metal salt of an organic acid. For example, at least a portion of the organic acid may be present in the oral product in the form of an alkali metal salt. Suitable alkali metal salts include lithium, sodium, and potassium. In some embodiments, the alkali metal is sodium or potassium. In some embodiments, the alkali metal is sodium. In some embodiments, the composition comprises an organic acid and a sodium salt of the organic acid.

In some embodiments, the oral product comprises benzoic acid and sodium benzoate, octanoic acid and sodium octanoate, decanoic acid and sodium decanoate, or a combination thereof. In some embodiments, the oral product comprises benzoic acid and sodium benzoate. In some embodiments, the oral product comprises sodium benzoate.

In some embodiments, the ratio of the organic acid to the sodium salt (or other alkali metal salt) of the organic acid is from about 0.1 to about 10, such as from about 0.1, about 0.25, about 0.3, about 0.5, about 0.75, or about 1, to about 2, about 5, or about 10. For example, in some embodiments, both an organic acid and the sodium salt thereof are added to the other components of the composition, wherein the organic acid is added in excess of the sodium salt, in equimolar quantities with the sodium salt, or as a fraction of the sodium salt. One of skill in the art will recognize that the relative amounts will be determined by the desired pH of the composition, as well as the desired ionic strength. For example, the organic acid may be added in a quantity to provide a desired pH level of the oral product, while the alkali metal (e.g., sodium) salt is added in a quantity to provide the desired extent of ion pairing. As one of skill in the art will understand, the quantity of organic acid (i.e., the protonated form) present in the oral product, relative to the alkali metal salt or conjugate base form present in the oral product, will vary according to the pH of the composition and the pKa of the organic acid, as well as according to the actual relative quantities initially added to the oral product.

The amount of organic acid or an alkali metal salt thereof present in the oral product, relative to the basic amine containing active ingredient (e.g., nicotine), may vary. Generally, as the concentration of the organic acid (or the conjugate base thereof) increases, the percent of nicotine that is ion paired with the organic acid increases. This typically increases the partitioning of the nicotine, in the form of an ion pair, into octanol versus water as measured by the logP (the log10 of the partitioning coefficient). In some embodiments, the composition comprises from about 0.05, about 0.1, about 1, about 1.5, about 2, or about 5, to about 10, about 15, or about 20 molar equivalents of the organic acid, the alkali metal salt thereof, or the combination thereof, relative to the nicotine component, calculated as free base nicotine.

In some embodiments, the oral product comprises from about 2 to about 10, or from about 2 to about 5 molar equivalents of the organic acid, the alkali metal salt thereof, or the combination thereof, to nicotine, on a free-base nicotine basis. In some embodiments, the organic acid, the alkali metal salt thereof, or the combination thereof, is present in a molar ratio with the nicotine from about 2, about 3, about 4, or about 5, to about 6, about 7, about 8, about 9, or about 10. In embodiments wherein more than one organic acid, alkali metal salt thereof, or both, are present, it is to be understood that such molar ratios reflect the totality of the organic acids present.

In some embodiments the organic acid inclusion is sufficient to provide a pH of the oral product from about 4.0 to about 9.5, such as from about 4.0 to about 9.0, or from about 4.0 to about 8.5, or from about 4.0 to about 8.0, or from about 4.5 to about 7.5, or from about 4.5 to about 7.0, or from about 5.5 to about 7.0, or from about 4.0 to about 5.5, or from about 7.0 to about 9.5. In some embodiments, the organic acid inclusion is sufficient to provide an oral product pH of about 4.0, about 4.5, about 5.0, about 5.5, about 6.0, about 6.5, about 7.0, about 7.5, about 8.0, about 8.5, or about 9.0. In some embodiments, the organic acid inclusion is sufficient to provide an oral product pH of from about 4.5 to about 6.5, for example, from about 4.5, about 5.0, or about 5.5, to about 6.0, or about 6.5. In some embodiments, the organic acid is provided in a quantity sufficient to provide a pH of the oral product of from about 5.5 to about 6.5, for example, from about 5.5, about 5.6, about 5.7, about 5.8, about 5.9, or about 6.0, to about 6.1, about 6.2, about 6.3, about 6.4, or about 6.5. In some embodiments, a mineral acid (e.g., hydrochloric acid, sulfuric acid, phosphoric acid, or the like) is added to adjust the pH of the oral product to the desired value. For avoidance of doubt, reference to the pH of the oral product refers to the pH of an aqueous solution prepared by immersing the product in a specified volume of water for a time sufficient to extract substantially all of the soluble components therein. Unless otherwise specified, the pH is determined by placing about 5 g of the relevant material (pouch, composition, or SEDS component) in about 50 mL of deionized water and stirring for 15 minutes.

In some embodiments, the organic acid is added as the free acid, either neat (i.e., native solid or liquid form) or as a solution in, e.g., water, to the other oral product components. In some embodiments, the alkali metal salt of the organic acid is added, either neat or as a solution in, e.g., water, to the other oral product components.

In some embodiments, the organic acid is added as the free acid, either neat (i.e., native solid or liquid form) or as a solution in, e.g., water, to the other oral product components. In some embodiments, the alkali metal salt of the organic acid is added, either neat or as a solution in, e.g., water, to the other composition components. In some embodiments, the organic acid and the basic amine (e.g., nicotine) are combined to form a salt, either before addition to the oral product, or the salt is formed within and is present in the oral product as such. In some embodiments, the organic acid and basic amine (e.g., nicotine) are present as individual components in the oral product and form an ion pair upon contact with moisture (e.g., saliva in the mouth of the consumer).

In some embodiments, the oral product comprises nicotine benzoate and sodium benzoate, wherein at least a portion of the nicotine and benzoate ions present are in an ion paired form. In some embodiments, the oral product comprises nicotine benzoate, sodium benzoate, and an organic acid, an alkali metal salt of an organic acid, or a combination thereof, the organic acid having a logP value from about 1 to about 12, wherein the organic acid is a monoester of a dicarboxylic acid or is a carotenoid derivative having one or more carboxylic acids.

In some embodiments, the oral product further comprises a solubility enhancer to increase the solubility of one or more of the organic acid or salt thereof. Suitable solubility enhancers include, but are not limited to, humectants as described herein, such as glycerol or propylene glycol.

In particular embodiments, the oral product comprises nicotine benzoate and sodium benzoate. In some embodiments, the sodium benzoate is present in a molar ratio to the nicotine benzoate in a range from about 1 to about 20, such as about 2, about 5, or about 10. In some embodiments, the oral product further comprises nicotine polacrilex. In some embodiments, the total amount of nicotine present in the oral product is provided in equal quantities from nicotine benzoate and nicotine polacrilex. In some embodiments, the oral product includes only nicotine polacrilex as the nicotine source, and the oral product optionally comprises an organic acid component such as sodium benzoate.

### Flavoring Agent

In some embodiments, the SEDS component, the oral product, or both include one or more flavoring agents. In some embodiments, one or more flavoring agents is present in the SEDS component.

In some embodiments, one or more flavoring agents are present in the composition comprising the oral product. In some embodiments, one or more flavoring agents are present in both the SEDS component and the composition comprising the oral product. The flavoring agents present in each may be the same or may be different.

As used herein, a "flavoring agent" or "flavorant" is any flavorful or aromatic substance capable of altering the sensory characteristics associated with the oral product. Examples of sensory characteristics that can be modified by the flavoring agent include taste, mouthfeel, moistness, coolness/heat, and/or fragrance/aroma. Flavoring agents may be natural or synthetic, and the character of the flavors imparted thereby may be described, without limitation, as fresh, sweet, herbal, confectionary, floral, fruity, or spicy. Specific types of flavors include, but are not limited to, vanilla, coffee, chocolate/cocoa, cream, mint, spearmint, menthol, peppermint, wintergreen, eucalyptus, lavender, cardamon, nutmeg, cinnamon, clove, cascarilla, sandalwood, honey, jasmine, ginger, anise, sage, licorice, lemon, orange, apple, peach, lime, cherry, strawberry, and any combinations thereof. See also, Leffingwell et al., Tobacco Flavoring for Smoking Products, R. J. Reynolds Tobacco Company (1972), which is incorporated herein by reference. Flavorings also may include components that are considered moistening, cooling or smoothening agents, such as eucalyptus. These flavors may be provided neat (i.e., alone) or in a composite, and may be employed as concentrates or flavor packages (e.g., spearmint and menthol, orange and cinnamon; lime, tropical, and the like). Representative types of components also are set forth in US Pat. No. 5,387,416 to White et al.; US Pat. App. Pub. No. 2005/0244521 to Strickland et al.; and PCT Application Pub. No. WO 05/041699 to Quinter et al., each of which is incorporated herein by reference. In some instances, the flavoring agent may be provided in a spray-dried form or a liquid form.

The flavoring agent generally comprises at least one volatile flavor component. As used herein, "volatile" refers to a chemical substance that forms a vapor readily at ambient temperatures (i.e., a chemical substance that has a high vapor pressure at a given temperature relative to a nonvolatile substance). Typically, a volatile flavor component has a molecular weight below about 400 and will often include at least one carbon-carbon double bond, carbon-oxygen double bond, carbon-oxygen single bond, or combinations thereof. In one embodiment, the at least one volatile flavor component comprises one or more alcohols, aldehydes, aromatic hydrocarbons, ketones, esters, terpenes, terpenoids, or a combination thereof. Non-limiting examples of aldehydes include vanillin, ethyl vanillin, p-anisaldehyde, hexanal, furfural, isovaleraldehyde, cuminaldehyde, benzaldehyde, and citronellal. Non-limiting examples of ketones include 1-hydroxy-2-propanone and 2-hydroxy-3-methyl-2-cyclopentenone-1-one. Non-limiting examples of terpenes include sabinene, limonene, gamma-terpinene, beta-farnesene, nerolidol, thujone, myrcene, geraniol, nerol, citronellol, linalool, and eucalyptol.

In some embodiments, the flavor comprises menthol, spearmint and/or peppermint. In some embodiments, the flavorant is lipophilic. In some embodiments, the flavoring agent comprises a terpene.

The amount of flavorant utilized can vary, but is typically up to about 10% by weight, and some embodiments are characterized by a flavoring agent content of at least about 0.1% by weight, such as about 0.1% to about 10% by weight, or about 1 to about 10% by weight, based on the total weight of the SEDS component.

### Solubilizer

The SEDS component comprises at least one solubilizer. By "solubilizer" is meant an emulsifier that is completely water soluble but has little oil solubility. As such, they are used to solubilize a lipid component (e.g., an oil) in water. Accordingly, one of skill in the art will understand that there may be some overlap between solubilizers and emulsifying agents (i.e., some emulsifiers may function as solubilizers, and some solubilizers may function more generally as emulsifying agents). Suitable solubilizers include, but are not limited to, hydrophilically modified fats or oils. In some embodiments, the at least one solubilizer comprises a glycerol monoester of a fatty acid. In some embodiments, the glycerol monoester of the fatty acid is glyceryl monooleate, glyceryl monolineolate, or a combination thereof.

### Emulsifying Agent

The SEDS component comprises at least one emulsifying agent. By "emulsifying agent" is meant a substance which aids in the formation and stabilization of emulsions by promoting dispersion of hydrophobic and hydrophilic (e.g., oil and water) components. In general, emulsifying agents are amphiphilic molecules chosen from, for example, nonionic and ionic amphiphilic molecules. The expression "amphiphilic molecule" means any molecule of bipolar structure comprising at least one hydrophobic portion and at least one hydrophilic portion and having the property of reducing the surface tension of water and of reducing the interface tension between water and an oily phase. Emulsifying agents/amphiphilic molecules as provided herein are also referred to as, for example, surfactants and emulsifiers.

Suitable emulsifying agents include small molecule surfactants, phospholipids, proteins, polysaccharides, and mixtures thereof. Examples of suitable emulsifying agents include, but are not limited to, polyethylene glycol esters of fatty acids, propylene glycol esters of fatty acids, polysorbates, polyglycerol esters of fatty acids, polyglycerol polyricinoleate, sorbitan esters of fatty acid, sucrose esters of fatty acids, lecithins, enzyme treated lecithins, glycerin fatty acids esters, acetic acid esters of monoglycerides, lactic acid esters of monoglycerides, citric acid esters of monoglycerides, succinic acid esters of monoglycerides, diacetyl tartaric acid esters of monoglycerides, calcium stearoyl di lactate, chitin and chitosan derivatives, natural and modified starches, natural and modified hydrocolloids, natural and modified polysaccharides, natural and modified celluloses, natural and modified proteins, synthetic amphiphilic polymers, glycol distearate, sorbitan trioleate, sorbitan tristearate, sorbitan triisostearate, glyceryl isostearate, propylene glycol isostearate, glycol stearate, sorbitan sesquioleate, glyceryl stearate, lecithin, sorbitan oleate, sorbitan monostearate, sorbitan stearate, sorbitan isostearate, steareth-2, oleth-2, PEG-7 hydrogenated castor oil, laureth-2, sorbitan palmitate, laureth-3, glyceryl laurate, ceteth-2, PEG-30 dipolyhdroxystearate, glyceryl stearate SE, sorbitan stearate (and) sucrose cocoate, PEG-4 dilaurate, methyl glucose sesquistearate, PEG-8 dioleate, sorbitan laurate, PEG-40 sorbitan peroleate, laureth-4, PEG-7 glyceryl cocoate, PEG-20 almond glycerides, PEG-25 hydrogenated castor oil, stearamide MEA, glyceryl stearate (and) PEG-100 stearate, polysorbate 81, polysorbate 85, polysorbate 65, PEG-7 glyceryl cocoate, PEG-8 stearate, PEG-8 caprate, PEG-35 almond glycerides, PEG-6 laurate, laureth-7, steareth-10, isotrideceth-8, PEG-35 castor oil, isotrideceth-9, PEG-40 castor oil, ceteareth-12, laureth-9, PEG-40 hydrogenated castor oil, PEG-20 glyceryl isostearate, PEG-20 stearate, PEG-40 sorbitan perisostearate, PEG-7 olivate, cetearyl glucoside, PEG-8 oleate, polyglyceryl-3 methylglucose distearate, oleth-10, oleth-10/polyoxyl 10 oleyl ether NF, ceteth-10, PEG-8 laurate, cocamide MEA, polysorbate 60, polysorbate 80, isosteareth-20, PEG-60 almond glycerides, PEG-20 methyl glucose sesquistearate, ceteareth-20, oleth-20, steareth-20, steareth-21, ceteth-20, isoceth-20, polysorbate 20, polysorbate 40, ceteareth-25, ceteareth-30, PEG-30 stearate, laureth-23, PEG-75 lanolin, polysorbate 20, PEG-40 stearate, PEG-100 stearate, steareth-100, PEG-80 sorbitan laurate, polyoxyethylene stearate (e.g., polyoxyethylene (40) stearate), polyoxyethylene ether, and mixtures thereof.

In some embodiments, the emulsifying agent has an HLB value in a range from about 1 to about 9. As will be understood by one skilled in the art, HLB is the hydrophilic-lipophilic balance of an emulsifying agent and is a measure of the degree to which it is hydrophilic or lipophilic. The HLB value may be determined by calculating values for the different regions of the molecule, as described by Griffin in Griffin, William C. (1949), "Classification of Surface-Active Agents by 'HLB'" (PDF), Journal of the Society of Cosmetic Chemists, 1 (5): 311-26 and Griffin, William C. (1954), "Calculation of HLB Values of Non-Ionic Surfactants" (PDF), Journal of the Society of Cosmetic Chemists, 5 (4): 249-56, and by Davies in Davies JT (1957), "A quantitative kinetic theory of emulsion type, I. Physical chemistry of the emulsifying agent" (PDF), Gas/Liquid and Liquid/Liquid Interface, Proceedings of the International Congress of Surface Activity, pp. 426-38. HLB value may be determined in accordance with the industry standard textbook, namely "The HLB SYSTEM, a time-saving guide to emulsifier selection" ICI Americas Inc., Published 1976 and Revised, March, 1980. The HLB values of the emulsifiers described herein were determined in accordance with this standard method.

Examples of suitable emulsifying agents having an HLB value in the range from about 1 to about 9 include, but are not limited to, mono and diglycerides of fatty acid including glyceryl stearate and glyceryl oleate; fatty acid esters of C12-C22 fatty alcohols including fatty acid esters of cetyl alcohol and fatty acid esters of stearoyl alcohol, mixtures of fatty acid esters of cetyl alcohol and fatty acid esters of stearoyl alcohol, mixtures of fatty acid esters of cetyl alcohol and fatty acid esters of stearoyl alcohol wherein the fatty acids are derived from olive oil (such as cetearyl olivate), fatty acid esters of sorbitol including sorbitan oleate, fatty acid esters of sorbitol wherein the fatty acids are derived from olive oil (such as sorbitan olivate or cetearyl olivate), and mixtures thereof. In some embodiments, the emulsifying agent is glycerol monolineolate, glycol distearate, sorbitan trioleate, sorbitan tristearate, sorbitan triisostearate, glyceryl isostearate, propylene glycol isostearate, glycol stearate, sorbitan sesquioleate, glyceryl stearate, lecithin (such as soy lecithin), sorbitan oleate, sorbitan monostearate, sorbitan stearate, sorbitan isostearate, steareth-2, oleth-2, polyethyleneglycol-7 (PEG-7) hydrogenated castor oil, laureth-2, sorbitan palmitate, laureth-3, glyceryl laurate, ceteth-2, PEG-30 dipolyhydroxystearate, glyceryl stearate SE, PEG-4 dilaurate, methyl glucose sesquistearate, PEG-8 dioleate, sorbitan laurate, PEG-40 sorbitan peroleate, or a mixture thereof.

In some embodiments, the at least one emulsifying agent comprises polyethoxylated castor oil, a polyethylene glycol monoester of a fatty acid, a polyethylene glycol diester of a fatty acid, or a combination thereof. In some embodiments, the emulsifying agent is a hydrogenated castor oil-polyethylene glycol polymer, such as a hydrogenated castor oil-PEG-40 polymer. An example of such a hydrogenated castor oil-PEG-40 polymer is Kolliphor^{®} RH40, available from BASF, Inc.

The amount of the emulsifying agent(s) present in the SEDS may vary. The amount of the emulsifying agent(s) may be in a range of up to about 90% by weight, for example from about 50% to about 90%, such as about 50%, about 55%, about 60%, about 65%, about 70%, about 75%, about 80%, about 85%, or about 90% by weight, based on the entirety of the SEDS.

As noted above, significant overlap exists between the functions of solubilizers and emulsifying agents, and certain components of the SEDS component as described herein may therefor serve more than one function. Accordingly, it is not intended that the function of such components be rigidly categorized and defined. For example, in some embodiments, the SEDS component may comprise multiple materials without attempt to define such materials by their function. In some such embodiments, the SEDS component comprises polyethoxylated castor oil; linoleic acid monoglyceryl ester; and a mixture of polyethylene glycol 6 mono- and diesters of oleic acid. In some embodiments, the SEDS component comprises from about 40 to about 60% by weight of polyethoxylated castor oil; from about 17 to about 25% by weight of linoleic acid monoglyceryl ester; and from about 17 to about 25% by weight of a mixture of polyethylene glycol-6 mono- and diesters of oleic acid; wherein each amount by weight is based on the total weight of the SEDS component.

### Antioxidant

In some embodiments, the SEDS component comprises an antioxidant or a combination of antioxidants. As used herein, the term "antioxidant" refers to a substance which prevents or suppresses oxidation by terminating free radical reactions and may delay or prevent some types of cellular damage. Antioxidants may be naturally occurring or synthetic. Naturally occurring antioxidants include those found in foods and botanical materials. Non-limiting examples of antioxidants include certain botanical materials, vitamins, polyphenols, and phenol derivatives.

Examples of botanical materials which are associated with antioxidant characteristics include without limitation acai berry, alfalfa, allspice, annatto seed, apricot oil, basil, bee balm, wild bergamot, black pepper, blueberries, borage seed oil, bugleweed, cacao, calamus root, catnip, catuaba, cayenne pepper, chaga mushroom, chervil, cinnamon, dark chocolate, potato peel, grape seed, ginseng, gingko biloba, Saint John's Wort, saw palmetto, green tea, black tea, black cohosh, cayenne, chamomile, cloves, cocoa powder, cranberry, dandelion, grapefruit, honeybush, echinacea, garlic, evening primrose, feverfew, ginger, goldenseal, hawthorn, hibiscus flower, jiaogulan, kava, lavender, licorice, marjoram, milk thistle, mints (menthe), oolong tea, beet root, orange, oregano, papaya, pennyroyal, peppermint, red clover, rooibos (red or green), rosehip, rosemary, sage, clary sage, savory, spearmint, spirulina, slippery elm bark, sorghum bran hi-tannin, sorghum grain hi-tannin, sumac bran, comfrey leaf and root, goji berries, gutu kola, thyme, turmeric, uva ursi, valerian, wild yam root, wintergreen, yacon root, yellow dock, yerba mate, yerba santa, bacopa monniera, withania somnifera, Lion's mane, and silybum marianum. Such botanical materials may be provided in fresh or dry form, essential oils, or may be in the form of an extracts. The botanical materials (as well as their extracts) often include compounds from various classes known to provide antioxidant effects, such as minerals, vitamins, isoflavones, phytoesterols, allyl sulfides, dithiolthiones, isothiocyanates, indoles, lignans, flavonoids, polyphenols, and carotenoids. Examples of compounds found in botanical extracts or oils include ascorbic acid, peanut endocarb, resveratrol, sulforaphane, beta-carotene, lycopene, lutein, co-enzyme Q, carnitine, quercetin, kaempferol, and the like. See, e.g., Santhosh et al., Phytomedicine, 12(2005) 216-220, which is incorporated herein by reference.

Non-limiting examples of other suitable antioxidants include citric acid, Vitamin E or a derivative thereof, vitamin C or a derivative thereof (including fatty acid esters) a tocopherol, epicatechol, epigallocatechol, epigallocatechol gallate, erythorbic acid, sodium erythorbate, 4-hexylresorcinol, theaflavin, theaflavin monogallate A or B, theaflavin digallate, phenolic acids, glycosides, quercitrin, isoquercitrin, hyperoside, polyphenols, catechols, resveratrols, oleuropein, hydroquinone, butylated hydroxyanisole (BHA), butylated hydroxytoluene (BHT), tertiary butylhydroquinones (TBHQs), such as mono- tertiary butylhydroquinone (MTBHQ) and di-tertiary butylhydroquinone (DTBHQ), sodium benzoate, and combinations thereof.

Typically, the antioxidant(s) will be lipophilic. In some embodiments, the antioxidant is a tocopherol, BHT, a TBHQ, vitamin E or a derivative thereof, citric acid, a fatty acid ester of vitamin C, or a combination thereof. In some embodiments, the antioxidant is a tocopherol.

The antioxidant is typically at a concentration of from about 0.001% w/w to about 10% by weight, such as, e.g., from about from about 0.001%, about 0.005%, about 0.01% w/w, about 0.05%, about 0.1%, or about 0.5%, to about 1%, about 2%, about 3 %, about 4%, about 5%, about 6%, about 7%, about 8%, about 9%, or about 10%, based on the total weight of the SEDS component. In some embodiments, the SEDS component comprises one or more antioxidants in a total amount from about 0.1 to about 1% by weight, such as from about 0.4 to about 0.6% by weight.

### Lipid component

In some embodiments, the SEDS component comprises a lipid component. The lipid component of the SEDS is generally an oil. Any suitable oil may be used, including petroleum-based (e.g., mineral oil) and natural or naturally derived oils (e.g., from plant materials or animal sources). Generally, the oil is a food-grade oil. Such oils include, but are not limited to, vegetable oils (e.g., acai oil, almond oil, amaranth oil, apricot oil, apple seed oil, argan oil, avocado oil, babassu oil, beech nut oil, ben oil, bitter gourd oil, black seed oil, black currant seed oil, borage seed oil, borneo tallow nut oil, bottle gourd oil, brazil nut oil, buffalo gourd oil, butternut squash seed oil, cape chestnut oil, canola oil, carob cashew oil, cocoa butter, cocklebur oil, coconut oil, corn oil, cothune oil, coriander seed oil, cottonseed oil, date seed oil, dika oil, egus seed oil, evening primrose oil, false flax oil, flaxseed oil, grape seed oil, grapefruit seed oil, hazelnut oil, hemp oil, kapok seed oil, kenaf seed oil, lallemantia oil, lemon oil, linseed oil, macadamia oil, mafura oil, marula oil, meadowfoam seed oil, mongongo nut oil, mustard oil, niger seed oil, nutmeg butter, okra seed oil, olive oil, orange oil, palm oil, palm stearin, papaya seed oil, peanut oil, pecan oil, perilla seed oil, persimmon seed oil, pequi oil, pili nut oil, pine nut oil, pistachio oil, pomegranate seed oil, poppyseed oil, pracaxi oil, prune kernel oil, pumpkin seed oil, quinoa oil, ramtil oil, rapeseed oil, rice bran oil, royle oil, sacha inchi oil, safflower oil, sapote oil, seje oil, sesame oil, shea butter, soybean oil, sunflower oil, taramira oil, tea seed oil, thistle oil, tigernut oil, tobacco seed oil, tomato seed oil, walnut oil, watermelon seed oil, wheat germ oil, and combinations thereof), animal oils (e.g., cattle fat, buffalo fat, sheep fat, goat fat, pig fat, lard, camel fat, tallow, liquid margarine, fish oil, fish liver oil, whale oil, seal oil, and combinations thereof), and mineral oils.

In some embodiments, the lipid component is a natural, food-grade oil. In some embodiments, the oil is a natural or naturally derived oil. In some embodiments, the oil comprises a long chain fatty acid or a medium chain fatty acid, such as the long chain fatty acid or medium chain fatty acid or one or more of a glycerol mono-, di-, or triester of either thereof (i.e., a monoacylglycerol, diacylglycerol, triacylglycerol, or a combination thereof, wherein the acyl group is a medium or long chain fatty acid).

As used herein, "medium chain fatty acid" refers to a carboxylic (CO₂H) acid having an aliphatic carbon chain of from about 6 to about 11 carbon atoms. The aliphatic carbon chain may be straight or branched. The aliphatic carbon chain may be saturated (i.e., having all sp3 carbon atoms), or may be unsaturated (i.e., having at least one site of unsaturation). As used herein, the term "unsaturated" refers to the presence of a carbon-carbon, sp² double bond in one or more positions within the aliphatic carbon chain. Unsaturated alkyl groups may be mono- or polyunsaturated. Representative medium chain fatty acids include, but are not limited to, caproic acid, caprylic acid, decanoic acid, and undecanoic acid.

As used herein, "long chain fatty acid" refers to a carboxylic (CO₂H) acid having an aliphatic carbon chain of from about 11 to about 21 carbon atoms. The aliphatic carbon chain may be straight or branched. The aliphatic carbon chain may be saturated (i.e., having all sp³ carbon atoms), or may be unsaturated (i.e., having at least one site of unsaturation). As used herein, the term "unsaturated" refers to the presence of a carbon-carbon, sp² double bond in one or more positions within the aliphatic carbon chain. Unsaturated alkyl groups may be mono- or polyunsaturated. Representative long chain fatty acids include, but are not limited to undecylic acid, undecanoic acid, lauric acid, tridecanoic acid, myristic acid, pentadecanoic acid, palmitic acid, margaric acid, stearic acid, nonadecanoic acid, arachidic acid, heneicosanoic acid, α-linolenic acid, stearidonic acid, eicosapentaenoic acid, cervonic acid, linoleic acid, linolelaidic acid, γ-linolenic acid, dihomo-γ-linolenic acid, and arachidonic acid.

In some embodiments, the oil comprises a triacylglycerol, wherein the acyl group is a medium chain fatty acid as described herein. In some embodiments, the oil comprises a triacylglycerol, wherein the acyl group is a medium chain fatty acid as described herein. Such medium chain fatty acid triacylglycerols may also be referred to herein as "medium chain triglycerides" or "MCTs." In some embodiments, the oil is enriched in MCT's, meaning the oil has a greater concentration of MCTs relative to other triglycerides, such as short or long chain triglycerides.

In some embodiments, the oil comprises a triacylglycerol, wherein the acyl group is a long chain fatty acid as described herein. Such long chain fatty acid triacylglycerols may also be referred to herein as "long chain triglycerides" or "LCTs." In some embodiments, the oil is enriched in LCT's, meaning the oil has a greater concentration of LCTs relative to other triglycerides, such as short or medium chain triglycerides. In particular embodiments, the LCTs comprise a relatively large percentage of unsaturated long chain fatty acids (e.g., oleic and/or linoleic acids). Without wishing to be bound by any particular theory, it is believed that LCT's, and particularly LCTs containing a relatively large percentage of unsaturated fatty acids, may aid in promoting lymphatic transport of cannabinoid molecules present in the SEDS. One non-limiting example of an oil rich in LCTs, and specifically unsaturated fatty acid LCTs, is sunflower oil.

In some embodiments, the oil comprises castor oil, corn oil, coconut oil, cod liver oil, evening primrose oil, cottonseed oil, palm oil, rice bran oil, sesame oil, rapeseed oil, canola oil, cocoa butter, linseed oil, olive oil, peanut oil, soybean oil, safflower oil, flaxseed oil, sunflower oil, olive oil, or a combination thereof. In some embodiments, the oil is olive oil.

When present, the amount of lipid component present within the SEDS can vary. In some embodiments, the SEDS comprises a lipid component in an amount of from about 1% to about 30% by weight, such as from about 20% to about 30% by weight, such as from about 20% to about 25% by weight, based on the total weight of the SEDS.

### Water

In some embodiments, the SEDS may comprise water. The water content of the SEDS may vary according to the desired properties of the oral product and the emulsion produced upon use of the oral product. In some embodiments, the water content of the SEDS is up to about 10% by weight, based on the total weight of the SEDS. In some embodiments, the SEDS is substantially free of water, meaning no water has been added beyond trace amounts which may be present in components of the SEDS. For example, the SEDS may contain less than about 1%, less than about 0.1%, less than about 0.01%, less than about 0.001%, or even 0% water by weight.

### Properties of the SEDS and Emulsion Therefrom

As described herein above, the self-emulsifying delivery system (SEDS) is configured to provide droplets comprising e.g., a flavorant, active ingredient, or both. In some embodiments, the SEDS component is at least partially in the form of a pre-emulsion comprising micelles. Upon exposure of the oral product comprising the SEDS to mouth conditions (e.g., moisture from saliva and optionally, mechanical chewing forces), the SEDS components interact with water in saliva and optionally within the gastrointestinal tract to produce an emulsion. Without wishing to be bound by theory, it is believed that the SEDS serves to protect the active ingredient, flavorant, or both from degradation or premature volatilization, while the emulsion produced upon use may aid in providing more rapid and/or complete delivery of the active ingredient, flavorant, or both to the consumer.

In some embodiments, the micelles of the SEDS have an average size (i.e., diameter) in a range from about 10 nm to about 1,000 nm. "Average particle size" is synonymous with D₅₀, meaning half of the population of particles has a particle size above this point, and half below. D₉₀ particle size distribution indicates that 90% of the particles (by number) have a Feret diameter below a certain size. The size of nanoparticles may be determined by quasi-electric light scattering (QELS) as described in Bloomfield, Ann. Rev. Biophys. Bioeng., 10:421-450 (1981), incorporated herein by reference. It may also be measured by correlation spectroscopy that analyzes the fluctuation in scattering of light due to Brownian motion, or by transmission electron microscopy (TEM). Unless otherwise indicated, particle size values reported herein are determined by light scattering using a nanoscale Zetasizer (Malvern Zetasizer; Malvern Panalytical Inc., 117 Flanders Road, Westborough, MA, USA).

In some embodiments, the D₅₀ particle size of the micelles is from about 100 nm to about 300 nm. In some embodiments, the D₅₀ particle size is about 100 nm, about 110 nm, about 120 nm, about 130 nm, about 140 nm, about 150 nm, about 160 nm, about 170 nm, about 180 nm, about 190 nm, about 200 nm, about 210 nm, about 220 nm, about 230 nm, about 240 nm, about 250 nm, about 260 nm, about 270 nm, about 280 nm, about 290 nm, or about 300 nm. Without wishing to be bound by theory, it is believed that droplets in this size range may be more readily absorbed through the intestinal lumen, may be available for lymphatic distribution, or both.

The emulsion produced upon use of the oral product generally comprises nano-scale particles (or nano-scale droplets) having an average particle size (D₅₀) of about 60 nm.

The emulsion as described herein may be characterized by reference to a polydispersity index. Polydispersity indicates the uniformity of droplet size in an emulsion. The higher the value of polydispersity, the lower will be the uniformity of droplet size. It may be defined as the ratio of standard deviation to mean droplet size and may be measured by spectrophotometric methods. In some embodiments, the emulsion has a polydispersity index of less than about 0.5.

The emulsion as described herein may be characterized by reference to zeta potential. As appreciated by one skilled in the art, zeta potential is the measure of the electrical charge on particle surface in colloidal dispersions, for example, the charge on the surface of a droplet in the emulsion. Zeta potential may be measured with a zeta analyzer, for example a Malvern nano-series ZS Zetasizer. In some embodiments, the zeta potential of the particles is from about -50 mV to about +50 mV. In some embodiments, the zeta potential of the particles is from about -50 mV, about -40 mV, about -30 mV, about -20 mV, about -10 mV, or about 0 mV, to about 10 mV, about 20 mV, about 30 mV, about 40 mV, or about 50 mV.

### Oral Product

In one aspect of the disclosure is provided an oral product comprising the SEDS as described herein. The oral product is configured for insertion into a user's mouth (i.e., oral cavity). Such oral products comprising the SEDS may further comprise components such as fillers, binders, carrier materials, flow aids, humectants, salts, buffers, additional active ingredients or flavors, or combinations of any thereof. Such additional components are further described herein below.

### Filler

The oral product as described herein may comprise one or more fillers. Fillers may fulfill multiple functions, such as enhancing certain organoleptic properties such as texture and mouthfeel, enhancing cohesiveness or compressibility of the product, and the like. In some cases, fillers can serve multiple functions, and therefore some components of the oral product can be considered both a filler and, for example, a flow aid, a sweetener, or a binder. Generally, fillers are porous particulate materials and are cellulose-based. For example, suitable fillers are any non-tobacco plant material or derivative thereof, including cellulose materials derived from such sources. Examples of cellulosic non-tobacco plant material include cereal grains (e.g., maize, oat, barley, rye, buckwheat, and the like), sugar beet (e.g., FIBREX^{®} brand filler available from International Fiber Corporation), bran fiber, and mixtures thereof. Non-limiting examples of derivatives of non-tobacco plant material include starches as described herein (e.g., from potato, wheat, rice, corn), natural cellulose, and modified cellulosic materials.

In some embodiments, the filler includes a non-tobacco plant material or a derivative thereof. Non-limiting examples of derivatives of non-tobacco plant material include starches (e.g., from potato, wheat, rice, corn), natural cellulose, and modified cellulosic materials. "Starch" as used herein may refer to pure starch from any source, modified starch, or starch derivatives. Starch is present, typically in granular form, in almost all green plants and in various types of plant tissues and organs (e.g., seeds, leaves, rhizomes, roots, tubers, shoots, fruits, grains, and stems). Starch can vary in composition, as well as in granular shape and size. Often, starch from different sources has different chemical and physical characteristics. A specific starch can be selected for inclusion in the oral product based on the ability of the starch material to impart a specific organoleptic property to the oral product. Starches derived from various sources can be used. For example, major sources of starch include cereal grains (e.g., rice, wheat, and maize) and root vegetables (e.g., potatoes and cassava). Other examples of sources of starch include acorns, arrowroot, arracacha, bananas, barley, beans (e.g., favas, lentils, mung beans, peas, chickpeas), breadfruit, buckwheat, canna, chestnuts, colacasia, katakuri, kudzu, malanga, millet, oats, oca, Polynesian arrowroot, sago, sorghum, sweet potato, quinoa, rye, tapioca, taro, tobacco, water chestnuts, and yams. Certain starches are modified starches. A modified starch has undergone one or more structural modifications, often designed to alter its high heat properties. Some starches have been developed by genetic modifications and are considered to be "genetically modified" starches. Other starches are obtained and subsequently modified by chemical, enzymatic, or physical means. For example, modified starches can be starches that have been subjected to chemical reactions, such as esterification, etherification, oxidation, depolymerization (thinning) by acid catalysis or oxidation in the presence of base, bleaching, transglycosylation and depolymerization (e.g., dextrinization in the presence of a catalyst), cross-linking, acetylation, hydroxypropylation, and/or partial hydrolysis. Enzymatic treatment includes subjecting native starches to enzyme isolates or concentrates, microbial enzymes, and/or enzymes native to plant materials, e.g., amylase present in corn kernels to modify corn starch. Other starches are modified by heat treatments, such as pregelatinization, dextrinization, and/or cold-water swelling processes. Certain modified starches include monostarch phosphate, distarch glycerol, distarch phosphate esterified with sodium trimetaphosphate, phosphate distarch phosphate, acetylated distarch phosphate, starch acetate esterified with acetic anhydride, starch acetate esterified with vinyl acetate, acetylated distarch adipate, acetylated distarch glycerol, hydroxypropyl starch, hydroxypropyl distarch glycerol, and starch sodium octenyl succinate.

Additional examples of potential fillers include maltodextrin, dextrose, lactose, calcium carbonate, calcium phosphate, sugar alcohols, and cellulosic materials. Combinations of fillers can also be used.

In some embodiments, the filler comprises one or more sugar alcohols. Sugar alcohols are polyols derived from monosaccharides or disaccharides that have a partially or fully hydrogenated form. Sugar alcohols have, for example, about 4 to about 20 carbon atoms and include erythritol, arabitol, ribitol, isomalt, maltitol, dulcitol, iditol, mannitol, xylitol, lactitol, sorbitol, and combinations thereof (e.g., hydrogenated starch hydrolysates).

In some embodiments, the filler comprises a cellulose material or a cellulose derivative, such as microcrystalline cellulose ("mcc"). The mcc may be synthetic or semi-synthetic, or it may be obtained entirely from natural celluloses. The mcc may be selected from the group consisting of AVICEL^{®} grades PH-100, PH-102, PH-103, PH-105, PH-112, PH-113, PH-200, PH-300, PH-302, VIVACEL^{®} grades 101, 102, 12, 20 and EMOCEL^{®} grades 50M and 90M, and the like, and mixtures thereof.

In some embodiments, the filler comprises or is an inorganic material. Examples of potential inorganic fillers include calcium carbonate, calcium phosphate, and bioceramic materials (e.g., porous hydroxyapatite).

The amount of filler can vary, but is typically greater than about 10%, and up to about 75% of the composition by weight, based on the total weight of the composition. A typical range of filler within the composition can be from about 10 to about 75% by total weight of the composition, for example, from about 10, about 15, about 20, about 25, or about 30, to about 35, about 40, about 45, or about 50% by weight (e.g., about 10 to about 50%, or about 25 to about 45% by weight). In some embodiments, the amount of filler is at least about 20% by weight, such as at least about 25%, or at least about 30%, or at least about 35%, or at least about 40%, based on the total weight of the composition. In some embodiments, the amount of filler within the composition can be from about 32 to about 48% by total weight of the composition.

In some embodiments, the oral product comprises an active ingredient, a flavorant, or both, each in addition to that which may be present in the SEDS component. In some embodiments, at least a portion of the active ingredient, the flavorant, or both is adsorbed in or on the filler.

### Binder

In some embodiments, the oral product comprises a binder. Combinations of binders can also be used. Binders generally function as thickening or gelling agents. Typical binders can be organic or inorganic, or a combination thereof. Examples of potential binders include cellulosic materials, cellulose derivatives (e.g., cellulose ethers), polymeric materials, gums, maltodextrin, sugars, and sugar alcohols.

In some embodiments, the binder comprises a cellulose derivative. In some embodiments, the cellulose derivative is a cellulose ether (including carboxyalkyl ethers), meaning a cellulose polymer with the hydrogen of one or more hydroxyl groups in the cellulose structure replaced with an alkyl, hydroxyalkyl, or aryl group. Non-limiting examples of such cellulose derivatives include methylcellulose, hydroxypropylcellulose ("HPC"), hydroxypropylmethylcellulose ("HPMC"), hydroxyethyl cellulose, and carboxymethylcellulose ("CMC"). In one embodiment, the cellulose derivative is one or more of methylcellulose, HPC, HPMC, hydroxyethyl cellulose, and CMC. In some embodiments, the cellulose derivative is HPC.

When present, the amount of binder utilized can vary, but is typically up to about 5% by weight, and some embodiments are characterized by a binder content of at least about 0.1% by weight, such as from about 1% to about 5% by weight, or about 2% to about 4% by weight, based on the total weight of the composition.

### Flow aids

If necessary for downstream processing of the oral product, such as granulation, mixing, or molding, a processing aid (i.e., a flow aid) can also be added to the composition in order to enhance flowability of the composition. In some cases, flow aids can serve multiple functions, and therefore some components of the oral product can be considered both as a flow aid, and e.g., a filler or carrier material. Exemplary flow aids include, but are not limited to, microcrystalline cellulose, silica, polyethylene glycol, stearic acid, calcium stearate, magnesium stearate, zinc stearate, sodium stearyl fumarate, canauba wax, and combinations thereof.

When present, a representative amount of flow aid may make up at least about 1 percent or at least about 5 percent of the total dry weight of the oral product. Typically, the amount of flow aid within the oral product will not exceed about 10 percent of the total dry weight of the composition. In some embodiments, the flow aid is present in an amount from about 6 to about 8% by weight of the composition.

### Carrier material

In some embodiments, the oral product comprises a carrier material. In some embodiments, at least a portion of the SEDS component is adsorbed in or on the carrier material. The nature of the carrier material is generally a porous, particulate material capable of absorbing or adsorbing the SEDS component. Suitable carrier materials may also function as fillers or flow aids, and conversely, fillers and flow aids may serve as carrier materials. Accordingly, suitable carrier materials may include materials described herein above as fillers and flow aids. Generally, suitable carrier materials have high surface areas (e.g., ~1000 m² g⁻¹), small particle sizes (e.g., such as in a range from about a few nanometers up to about 1000 nanometers), and a small pore size (e.g., such as in a range from about a few nanometers up to about 500 nanometers). In some embodiments, the carrier is a mesoporous material. In some embodiments, the carrier material comprises microcrystalline cellulose, silica, a starch, maltodextrin, hydroxypropylmethyl cellulose, polyethylene glycol, stearic acid, calcium stearate, magnesium stearate, zinc stearate, sodium stearyl fumarate, calcium phosphate, calcium carbonate, a clay, talc, a hydrogentated vegetable oil, or a combination thereof. In some embodiments, the carrier material is silica or microcrystalline cellulose. In some embodiments, the carrier material is silica, and the entirety of the SEDS component is adsorbed in or on the silica.

### Sweetener

In order to improve the sensory and/or physical properties of the oral product, one or more sweeteners may be added. The sweeteners can be any sweetener or combination of sweeteners, in natural or artificial form, or as a combination of natural and artificial sweeteners. Examples of natural sweeteners include fructose, sucrose, glucose, maltose, isomaltulose, mannose, galactose, lactose, stevia, honey, and the like. Examples of artificial sweeteners include sucralose, maltodextrin, saccharin, aspartame, acesulfame K, neotame and the like. In some embodiments, the sweetener is selected from the group consisting of fructose, sucrose, glucose, maltose, mannose, galactose, lactose, stevia, honey, sucralose, isomaltulose, maltodextrin, saccharin, aspartame, acesulfame K, neotame, erythritol, arabitol, ribitol, isomalt, maltitol, dulcitol, iditol, mannitol, xylitol, lactitol, sorbitol, and mixtures thereof. In some embodiments, the sweetener is selected from the group consisting of maltitol, xylitol, acesulfame K, aspartame, and mixtures thereof.

In some embodiments, the sweetener comprises one or more sugars. Suitable sugars include, but are not limited to, glucose, sucrose, fructose, maltose, isomaltulose, mannose, galactose, lactose.

In some embodiments, the sweetener comprises one or more sugar alcohols. Sugar alcohols are polyols derived from monosaccharides or disaccharides that have a partially or fully hydrogenated form. Sugar alcohols have, for example, about 4 to about 20 carbon atoms and include erythritol, arabitol, ribitol, isomalt, maltitol, dulcitol, iditol, mannitol, xylitol, lactitol, sorbitol, and combinations thereof (e.g., hydrogenated starch hydrolysates). Sugar alcohols also provide certain characteristics to the oral product, for example, providing bulk, and in combination with other components (e.g., binder), provide physical attributes such as chewiness, firmness, softness, and the like. In some embodiments, the oral product is in the form of a chew, and comprises at least one sugar, at least one sugar alcohol, or a combination thereof. In some embodiments, the oral product in the form of a chew comprises at least one sugar alcohol. In some embodiments, the sugar alcohol is maltitol.

When present in the oral product, the sweetener or mixture of sweeteners may be present in an amount from about 0.1% to about 10% by weight of the composition, such as about 1 to about 5%, or about 1 to about 3% by weight, based on the total weight of the composition.

### Salt

In some embodiments, the oral product comprises a salt (e.g., an alkali metal salt), typically employed in an amount sufficient to provide desired sensory attributes to the product. Non-limiting examples of suitable salts include sodium chloride, potassium chloride, ammonium chloride, flour salt, sodium acetate, sodium citrate, and the like.

When present, a representative amount of salt is at least about 0.5% by weight, such as at least about 1% by weight, such as at least about 1.5% by weight. In some embodiments, the composition may comprise a salt in an amount of from about 0.5% to about 10% by weight, such as from about 1% to about 7.5% by weight, such as from about 1.5% to about 5% by weight, or from about 2 to about 3% by weight, based on the total weight of the composition.

### Sensates

In some embodiments, the oral product comprises a sensate. Sensates are compounds which chemically induce somatosensorial sensation through stimulation of the fifth cranial nerve (trigeminal nerve), in addition to or in place of aroma or taste nerves. Sensates include agents providing warming/heating, cooling, tingling, and numbing effects by interacting with (e.g., stimulating) the trigeminal nerve. For avoidance of doubt, such sensates are different and distinct from the flavoring agents disclosed herein.

Suitable sensates which provide to the user a warming effect include, but are not limited to, vanillyl alcohol n-butyl ether, vanillyl alcohol n-propylether, vanillyl alcohol isopropyl ether, vanillyl alcohol isobutyl ether, vanillyl alcohol n-aminoether, vanillyl alcohol isoamyl ether, vanillyl alcohol n-hexyle ther, vanillyl alcohol methyl ether, vanillyl alcohol ethyl ether, gingerol, shogaol, paradol, zingerone, capsaicin, dihydrocapsaicin, nordihydrocapsaicin, homocapsaicin, homodihydrocapsaicin, ethanol, isopropol alcohol, iso-amylalcohol, benzyl alcohol, glycerin, and combinations thereof. In some embodiments, the warming agent comprises vanillyl ethyl ether, capsaicin, or a combination thereof.

Suitable sensates which provide to the user a cooling effect include, but are not limited to, menthane, menthone, menthone ketals, menthone glycerol ketals, substituted p-menthanes, acyclic carboxamides, monomenthyl glutarate, substituted cyclohexanamides, substituted cyclohexane carboxamides, substituted ureas and sulfonamides, substituted menthanols, hydroxymethyl and hydroxymethyl derivatives of p-menthane, 2-mercapto-cyclo-decanone, hydroxycarboxylic acids with 2-6 carbon atoms, cyclohexanamides, menthyl acetate, menthyl salicylate, N,2,3-trimethyl-2-isopropyl butanamide (WS-23), N-ethyl-2,2-diisopropylbutanamide, N-ethyl-5-methyl-2-(1-methylethyl)-cyclohexane carboxamide (WS-3), N-[(ethoxycarbonyl)methyl)-p-menthane-3-carboxamide (WS-5), Evercool^{™} 180 ((1R,2S,SR)-N-(4-(cyanomethyl)phenyl)menthylcarboxamide), Evercool^{™} 190 ((1R,2S,SR)-N-(2-(pyridin-2-yl)ethyl)menthylcarboxamide), the substantially pure ethyl ester of N-[[5-methyl-2-(1-methylethyl)cyclohexyl]carbonyl]glycine (as disclosed in U.S. Pat. No. 7,189,760 to Erman, et al., which is incorporated in its entirety herein by reference), isopulegol, menthyloxy propanediol, 3-(1-menthoxy)propane-1,2-diol, 3-(1-menthoxy)-2-methylpropane-1,2-diol, p-menthane-2,3-diol, p-menthane-3,8-diol, 6-isopropyl-9-methyl-1,4-dioxaspiro[4,5]decane-2-methanol, menthyl succinate and its alkaline earth metal salts, trimethylcyclohexanol, N-ethyl-2-isopropyl-5-methylcyclohexanecarboxamide, Japanese mint oil, peppermint oil, 3-(1-menthoxy)ethan-1-ol, 3-(1-menthoxy)propan-1-ol, 3-(1-menthoxy)butan-1-ol, 1-menthylacetic acid N-ethylamide, 1-menthyl-4-hydroxypentanoate, 1-menthyl-3-hydroxybutyrate, N-2,3-trimethyl-2-(1-methylethyl)-butanamide, ethyl (E,Z)-2,6-nonadienamide, N,N-dimethyl menthyl succinamide, substituted p-menthanes, substituted p-menthane-carboxamides, 2-isopropanyl-5-methylcyclohexanol, menthone glycerol ketals, 3-1-menthoxypropane-1,2-diol, menthyl lactate, WS-30, WS-14, eucalyptus extract, menthol (including natural or synthetic derivatives), menthol PG carbonate, menthol EG carbonate, menthol glyceryl ether, N-tert-butyl-p-menthane-3-carboxamide, p-menthane-3-carboxylic acid glycerol ester; menthol methyl ether, menthyl pyrrolidone carboxylate, 2,5-dimethyl-4-(1-pyrrolidinyl)-3(2H)-furanone, cyclic a-keto enamines, and cyclopentenes (including 3-methyl-2-(1-pyrrolidinyl)-2-cyclopenten-1-one and 5-methyl-2-(1-pyrrolidinyl)-2-cyclopenten-1-one). Other suitable sensates which provide to the user a cooling effect include the alpha-keto enamines disclosed in U.S. Pat. No. 6,592,884 to Hofmann et al., which is incorporated in its entirety herein. These and other suitable cooling agents are further described in U.S. Pat. Nos. 4,230,688, 4,032,661, 4,459,425, 4,178,459, 4,296,255, 4,136,163, 5,009,893, 5,266,592, 5,698,181, 6,277,385, 6,627,233, 7,030,273, each of which are incorporated in their entirety by reference hereto. Still other suitable cooling agents are described in U.S. Patent Application Nos. 2005/0222256 and 2005/0265930, each of which is incorporated in their entirety by reference hereto. In some embodiments, the cooling agent comprises menthol, eucalyptus, mint, menthol, menthyl esters, eucolyptol, WS-3, WS-23, WS-5, Evercool^{™} 180 ((1R,2S,5R)-N-(4-(cyanomethyl)phenyl)menthylcarboxamide), Evercool^{™} 190 ((1R,2S,5R)-N-(2-(pyridin-2-yl)ethyl)menthylcarboxamide), or a combination thereof.

Suitable sensates which provide to the user a tingling, stinging, or numbing effect include, but are not limited to Jambu Oleoresin or Paracress (*Spilanthes* sp.), in which the active ingredient is Spilanthol; alkylamides extracted from materials such as jambu or sanshool; Japanese pepper extract (*Zanthoxylum peperitum*), including the ingredients known as Saanshool-I, Saanshool-II and Sanshoamide; perillartine; 4-(1-menthoxymethyl)-2-phenyl-1,3-dioxolane; black pepper extract (*Piper nigrum*), including the active ingredients chavicine and piperine; Echinacea extract; Northern Prickly Ash extract; trans-pellitorin, and red pepper oleoresin; and one or more sensory agents derived from Sichuan pepper (referred to herein as "Sichuan," "Sichuan pepper," or "Sichuan peppercorn"). Sichuan pepper can alternatively be referred to, e.g., as "Szechuan" pepper "Schezwan" pepper, Chinese prickly ash, Chinese pepper, Huajiao, rattan pepper, and mala pepper. Sichuan pepper is not actually a pepper; rather, it is the outer layer (coat/husk) of a berry, also referred to as "pericarps", from plants of the genus *Zanthoxylum* in the plant family Rutaceae and can be, e.g., red or green in color. This genus/family includes at least five species, including, *e.g*., *Zanthoxylum bungeanum* and *Zanthoxylum armatum*. Different species can differ somewhat in their exact composition and even the same species can differ from plant to plant, depending e.g., upon growing conditions. As such, the exact characteristics and extent of such characteristics provided by the inclusion of sensory agents derived from Sichuan pepper may vary. Further examples of "tingling" type sensates include those disclosed in U.S. Pat. Nos. 6,780,443, 6,159,509, 5,545,424, and 5,407,665, each of which is incorporated by reference herein in its entirety.

### Taste modifier

In some embodiments, the oral product comprises a taste modifying agent. In some embodiments, the taste modifier may mask the bitterness of the active ingredient, for example, a cannabinoid, in the oral product. The taste modifying agent may improve the organoleptic properties of an oral product as disclosed herein, and may serve to mask, alter, block, or improve e.g., the flavor of an oral product as disclosed herein. Non-limiting examples of such taste modifiers include but are not limited to analgesic or anesthetic herbs and spices. Certain taste modifiers fall into more than one overlapping category.

In some embodiments, the taste modifier modifies one or more of bitter, sweet, salty, or sour tastes. In some embodiments, the taste modifier targets pain receptors. In some embodiments, the oral product comprises a taste modifier which masks or blocks the perception of a bitter taste. In some embodiments, the taste modifier is a substance which targets pain receptors (e.g., vanilloid receptors) in the user's mouth to mask e.g., a bitter taste of another component. In some embodiments, the taste modifier is capsaicin.

In some embodiments, the taste modifier is the amino acid gamma-amino butyric acid (GABA), referenced herein above with respect to amino acids. Studies in mice suggest that GABA may serve function(s) in taste buds in addition to synaptic inhibition. See, e.g., Dvoryanchikov et al., J Neurosci. 2011 Apr 13;31(15):5782-91. Without wishing to be bound by theory, GABA may suppress the perception of certain tastes, such as bitterness.

In some embodiments, the taste modifier is adenosine monophosphate (AMP). AMP is a naturally occurring nucleotide substance which can block bitter food flavors or enhance sweetness. It does not directly alter the bitter flavor but may alter human perception of "bitter" by blocking the associated receptor.

In some embodiments, the taste modifier is lactisole. Lactisole is an antagonist of sweet taste receptors. Temporarily blocking sweetness receptors may accentuate e.g., savory notes.

When present, a representative amount of taste modifier is about 0.01% by weight or more, about 0.1% by weight or more, or about 1.0% by weight or more, but will typically make up less than about 10% by weight of the total weight of the composition, (e.g., from about 0.01%, about 0.05%, about 0.1%, or about 0.5%, to about 1%, about 5%, or about 10% by weight of the total weight of the composition).

### Humectant

In some embodiments, one or more humectants may be employed in the oral product of the present disclosure, and may be present in the SEDS, the overall oral product, or both. Examples of humectants include, but are not limited to, glycerin, 1,2-propanediol (propylene glycol), 1,3-propanediol, dipropylene glycol, sorbitol, xylitol, mannitol, and the like. In some embodiments, the oral product comprises glycerin. In some embodiments, the oral product comprises propylene glycol. In some embodiments, the oral product is substantially free of humectants.

Where included, the humectant is typically provided in an amount sufficient to provide desired moisture attributes to the oral product. When present, the humectant (such as glycerin and/or propylene glycol) may be present in an amount of from about 0.1% to about 15% by weight of the composition, such as from about 1 to about 12%, or from about 7 to about 11% by weight of the composition.

### Buffering Agent

In some embodiments, the oral product may comprise pH adjusters or buffering agents. Examples of pH adjusters and buffering agents that can be used include, but are not limited to, metal hydroxides (e.g., alkali metal hydroxides such as sodium hydroxide and potassium hydroxide), and other alkali metal buffers such as metal carbonates (e.g., potassium carbonate or sodium carbonate), or metal bicarbonates such as sodium bicarbonate, and the like. Non-limiting examples of suitable buffers include alkali metals acetates, glycinates, phosphates, glycerophosphates, citrates, carbonates, hydrogen carbonates, borates, or mixtures thereof. In some embodiments, the buffering agent is selected from the group consisting of sodium citrate, sodium carbonate, sodium bicarbonate, sodium phosphate, ammonium phosphate, and mixtures thereof.

Where present, the buffering agent is typically present in an amount less than about 5% based on the weight of the composition; for example, from about 0.1% to about 5%, such as, e.g., from about 0.1% to about 1%, or from about 0.5% to about 0.5%, or from about 0.2% to about 0.3% by weight, based on the total weight of the composition.

### Colorants

A colorant may be employed in amounts sufficient to provide the desired physical attributes to the oral product. Natural or synthetic colorants, such as natural or synthetic dyes, food-grade colorants and pharmaceutical-grade colorants may be used. Examples of colorants include various dyes and pigments, such as caramel coloring and titanium dioxide. Natural colorants such as curcumin, beet juice extract, spirulina may be used. Also, a variety of synthetic pigments may be used. In some embodiments, the colorant is a lake dye, such as a red or blue aluminum lake dye. The amount of colorant utilized in the oral product can vary, but when present is typically up to about 3% by weight, such as from about 0.1%, about 0.5%, or about 1%, to about 3% by weight, based on the total weight of the composition.

### Oral care additives

In some embodiments, the oral product comprises an oral care ingredient (or mixture of such ingredients). Oral care ingredients provide the ability to inhibit tooth decay or loss, inhibit gum disease, relieve mouth pain, whiten teeth, or otherwise inhibit tooth staining, elicit salivary stimulation, inhibit breath malodor, freshen breath, or the like. For example, effective amounts of ingredients such as thyme oil, eucalyptus oil and zinc (e.g., such as the ingredients of formulations commercially available as ZYTEX^{®} from Discus Dental) can be incorporated into the composition. Other examples of ingredients that can be incorporated in desired effective amounts within the present composition can include those that are incorporated within the types of oral care compositions set forth in Takahashi et al., Oral Microbiology and Immunology, 19(1), 61-64 (2004); U.S. Pat. No. 6,083,527 to Thistle; and US Pat. Appl. Pub. Nos. 2006/0210488 to Jakubowski and 2006/02228308 to Cummins et al. Other exemplary ingredients include those contained in formulations marketed as MALTISORB^{®} by Roquette and DENTIZYME^{®} by NatraRx. When present, a representative amount of oral care additive is at least about 1%, often at least about 3%, and frequently at least about 5% of the total dry weight of the composition. The amount of oral care additive within the oral product will not typically exceed about 30%, often will not exceed about 25%, and frequently will not exceed about 20%, of the total dry weight of the composition.

### Other Additives

Other additives can be included in the oral product. For example, the oral product can be processed, blended, formulated, combined, and/or mixed with other materials or ingredients. The additives can be artificial or can be obtained or derived from herbal or biological sources. Examples of further types of additives include thickening or gelling agents (e.g., fish gelatin), preservatives (e.g., potassium sorbate, sodium benzoate, calcium propionate, and the like), disintegration aids, zinc or magnesium salts selected to be relatively water soluble for oral products with greater water solubility (e.g., magnesium or zinc gluconate) or selected to be relatively water insoluble for oral products with reduced water solubility (e.g., magnesium or zinc oxide), or combinations thereof. See, for example, those representative components, combination of components, relative amounts of those components, and manners and methods for employing those components, set forth in US Pat. No. 9,237,769 to Mua et al., US Pat. No. 7,861,728 to Holton, Jr. et al., US Pat. App. Pub. No. 2010/0291245 to Gao et al., and US Pat. App. Pub. No. 2007/0062549 to Holton, Jr. et al., each of which is incorporated herein by reference.

Typical inclusion ranges for such additional additives can vary depending on the nature and function of the additive and the intended effect on the final product, with an example range of up to about 10% by weight, (e.g., from about 0.1% to about 5% by weight) based on total weight of the composition. For example, where present, a preservative (such as potassium sorbate, sodium benzoate, calcium propionate, or the like) can be included in the oral product in an amount of from about 0.001% to about 5% by weight of the composition, such as from about 0.01% to about 2.5% by weight of the oral product, such as from about 0.05% to about 1% by weight of the composition.

### Configured for Oral Use

The oral products as described herein are configured for oral use. The term "configured for oral use" as used herein means that the product is provided in a form such that during use, saliva in the mouth of the user causes one or more of the components of the product (e.g., flavoring agents and/or active ingredients) to pass into the mouth of the user. In some embodiments, the emulsion produced upon use of the product is adapted to deliver components to a user through mucous membranes in the user's mouth, the user's digestive system, or both, and, in some instances, said component is an active ingredient that can be absorbed through the mucous membranes in the mouth or absorbed through the digestive tract when the product is used.

Compositions as described herein may take various forms, including pellets, tablets, granules, beads, and powders, any of which may be contained within a pouch. The compositions as disclosed herein can be formed into a variety of shapes, including pills, tablets, spheres, beads, ovoids, obloids, and the like. Cross-sectional shapes of the composition can vary, and example cross-sectional shapes include circles, squares, ovals, rectangles, and the like. Such shapes can be formed in a variety of manners using equipment such as moving belts, nips, extruders, granulation devices, compaction devices, and the like. In some embodiments, oral products configured for oral use as described herein comprise the composition of the present disclosure (i.e., comprising a SEDS component) disposed within a moisture-permeable container (e.g., a water-permeable pouch). In some embodiments, the composition is in particulate form (e.g., powder or granular) enclosed within a pouch. Such compositions in the water-permeable pouch format are typically used by placing one pouch containing the composition in the mouth of a human subject/user. Generally, the pouch is placed somewhere in the oral cavity of the user, for example under the lips, in the same way as moist snuff products are generally used. The pouch generally is not chewed or swallowed. Exposure to saliva then causes an emulsion to form from the SEDS component. Some of the components of the SEDS component and/or the composition comprising the SEDS (e.g., flavoring agents and/or active ingredients) to pass through e.g., the water-permeable pouch and provide the user with flavor and satisfaction. After about 5 minutes to about 60 minutes, typically about 10 minutes to about 45 minutes of use/enjoyment, substantial amounts of the desired components have been released and absorbed through oral mucosa of the human subject, and the pouch may be removed from the mouth of the human subject for disposal.

Some embodiments of the disclosure will be described with reference to the accompanying drawing, and these described embodiments involve snus-type products having an outer pouch and containing a composition as described herein. As explained in greater detail below, such embodiments are provided by way of example only, and the pouched products of the present disclosure can include the composition in other forms. The composition/construction of such packets or pouches, such as the container pouch **102** in the embodiment illustrated in the drawing may be varied. Referring to the drawing, there is shown a first embodiment of a pouched product **100.** The pouched product **100** includes a moisture-permeable container in the form of a pouch **102,** which contains a composition **104** as described herein. Pouches as described herein have three dimensions: a length, a width, and a thickness. One of skill in the art will recognize that such dimensions may vary depending on the intended overall size and volume of the pouch, and the quantity of material desired within the pouch.

The type of pouch may vary. The pouch provides a liquid-permeable container of a type that may be considered to be similar in character to the mesh-like type of material that is used for the construction of a tea bag. Components of the composition readily diffuse through the pouch and into the mouth of the user.

Non-limiting examples of suitable types of pouches are set forth in, for example, US Pat. Nos. 5,167,244 to Kjerstad and 8,931,493 to Sebastian et al.; as well as US Patent App. Pub. Nos. 2016/0000140 to Sebastian et al.; 2016/0073689 to Sebastian et al.; 2016/0157515 to Chapman et al.; and 2016/0192703 to Sebastian et al., each of which is incorporated herein by reference. Pouches can be provided as individual pouches, or a plurality of pouches (e.g., 2, 4, 5, 10, 12, 15, 20, 25 or 30 pouches) can be connected or linked together (e.g., in an end-to-end manner) such that a single pouch or individual portion can be readily removed for use from a one-piece strand or matrix of pouches.

The pouches of the present disclosure can be formed from a fleece material, e.g., fibrous nonwoven webs. As used herein, the term "fiber" is defined as a basic element of textiles. Fibers are often in the form of a rope- or string-like element. As used herein, the term "fiber" is intended to include fibers, filaments, continuous filaments, staple fibers, and the like. The term "multicomponent fibers" refers to fibers that comprise two or more components that are different by physical or chemical nature, including bicomponent fibers. Specifically, the term "multicomponent fibers" includes staple and continuous fibers prepared from two or more polymers present in discrete structured domains in the fiber, as opposed to blends where the domains tend to be dispersed, random or unstructured.

A "fleece material" as used herein may be formed from various types of fibers, as described in more detail herein below, capable of being formed into a traditional fleece fabrics or other traditional pouch materials. For example, fleece materials may be provided in the form of a woven or nonwoven fabric. Suitable types of fleece materials, for example, are described in U.S. Patent No. 8,931,493 to Sebastian et al.; and US Patent App. Pub. Nos. 2015/0128978 to Sebastian et al., 2016/0000140 to Sebastian et al., and US Patent App. Pub. No. 2016/0073689 to Sebastian et al.; which are all incorporated herein by reference.

The term "nonwoven" is used herein in reference to fibrous materials, webs, mats, batts, or sheets in which fibers are aligned in an undefined or random orientation. The nonwoven fibers are initially presented as unbound fibers or filaments. An important step in the manufacturing of nonwovens involves binding the various fibers or filaments together. The manner in which the fibers or filaments are bound can vary, and include thermal, mechanical and chemical techniques that are selected in part based on the desired characteristics of the final product, as discussed in more detail herein below.

In some embodiments, the pouch may be biodegradable or dissolvable. In some embodiments, after use, the entire composition, and in some embodiments, the entirety of the pouch material originally housing the composition, can be dissolved and orally ingested by the user such that there is nothing left of the pouched product to remove from the mouth of the user. An example dissolvable pouch may be manufactured from materials, and in such a manner, such that during use by the user, the pouch undergoes a controlled dispersion or dissolution. Such pouch materials may have the form of a mesh, screen, perforated paper, permeable fabric, or the like. For example, pouch material manufactured from a mesh-like form of rice paper, or perforated rice paper, may dissolve in the mouth of the user. As a result, the pouch and composition each may undergo complete dispersion within the mouth of the user during normal conditions of use, and hence the pouch and composition both may be ingested by the user. In various embodiments, the pouch material can be dissolvable (i.e., orally ingestible) such that under conditions of normal use (i.e., upon contact with saliva in the mouth of a user), the pouch material completely dissolves. Typically, the pouch material will dissolve after a significant amount of the soluble components of the composition within the pouch (e.g., active ingredient(s) and/or flavorant(s)) permeate through the pouch material into the mouth of the user. For example, the pouch material can be configured to dissolve at a rate such that the pouch material holds the composition together for a period of time sufficient to allow for the release of substantially all water-soluble components.

A pouched product as described herein can be packaged within any suitable inner packaging material and/or outer container. See also, for example, the various types of containers set forth in US Pat. Nos. 7,014,039 to Henson et al.; 7,537,110 to Kutsch et al.; 7,584,843 to Kutsch et al.; 8,397,945 to Gelardi et al., D592,956 to Thiellier; D594,154 to Patel et al.; and D625,178 to Bailey et al.; US Pat. Pub. Nos. 2008/0173317 to Robinson et al.; 2009/0014343 to Clark et al.; 2009/0014450 to Bjorkholm; 2009/0250360 to Bellamah et al.; 2009/0266837 to Gelardi et al.; 2009/0223989 to Gelardi; 2009/0230003 to Thiellier; 2010/0084424 to Gelardi; and 2010/0133140 to Bailey et al; 2010/0264157 to Bailey et al.; and 2011/0168712 to Bailey et al. which are incorporated herein by reference.

The amount of material contained within each pouch may vary. In some embodiments, the weight of the composition within each pouch is at least about 50 mg, for example, from about 50 mg to about 2 grams, from about 100 mg to about 1.5 grams, or from about 200 to about 700 mg. In some smaller embodiments, the weight of the composition within each pouch may be from about 100 to about 300 mg. For a larger embodiment, the weight of the material within each pouch may be from about 300 mg to about 700 mg.

If desired, other components can be contained within each pouch. For example, at least one flavored strip, piece or sheet of flavored water dispersible or water-soluble material (e.g., a breath-freshening edible film type of material) may be disposed within each pouch along with or without at least one capsule. Such strips or sheets may be folded or crumpled in order to be readily incorporated within the pouch. See, for example, the types of materials and technologies set forth in US Pat. Nos. 6,887,307 to Scott et al. and 6,923,981 to Leung et al.; and The EFSA Journal (2004) 85, 1-32; which are incorporated herein by reference. In some embodiments, the pouch contents further comprise microcrystalline cellulose as described herein above.

While the compositions described herein have generally been described as disposed within a pouch, products of the present disclosure are not limited to such pouched embodiments. Accordingly, any embodiment of the disclosed composition may also be used in the absence of a pouch. For instance, a composition as described herein may simply be applied directly to the oral cavity as e.g., a powder, granules, beads, tablets, pellets, or the like.

### Preparation of the oral product

In a general, non-limiting embodiment, oral products of the disclosure are prepared by preparing a SEDS component, combining the SEDS component with other components forming the composition, and enclosing the composition in a pouch.

Generally, the SEDS component is prepared by combining at least one solubilizer, at least one emulsifying agent, an antioxidant, and optionally, a lipid component, each as described herein above. The combining is generally performed by mixing the components by any suitable means, such as stirring, to form the SEDS component.

In some embodiments, the SEDS component is adsorbed in or on a carrier material, such as materials characterized herein as fillers or flow aids. Accordingly, in some embodiments, the SEDS component is contacted with a carrier material (e.g., silica) to form an adsorbed SEDS component. Such an adsorbed SEDS component was found according to the present disclosure to ease subsequent processing. Thus, the carrier material may be referred to as a flow aid in view of its function.

The SEDS component, optionally adsorbed on a carrier material, is then combined with the desired composition components. For example, in some embodiments, the SEDS component is combined with a filler; an active ingredient, a flavoring agent, or a combination thereof; and optionally, one or more sweeteners, one or more humectants, one or more binding agents, one or more salts, one or more buffering agents, or a combination thereof. These various components may be contacted, combined, or mixed together using any mixing technique or equipment known in the art. Any mixing method that brings the oral product ingredients into intimate contact can be used, such as a mixing apparatus featuring an impeller or other structure capable of agitation. Examples of mixing equipment include casing drums, conditioning cylinders or drums, liquid spray apparatus, conical-type blenders, ribbon blenders, mixers available as FKM130, FKM600, FKM1200, FKM2000 and FKM3000 from Littleford Day, Inc., Plough Share types of mixer cylinders, Hobart mixers, and the like. See also, for example, the types of methodologies set forth in US Pat. Nos. 4,148,325 to Solomon et al.; 6,510,855 to Korte et al.; and 6,834,654 to Williams, each of which is incorporated herein by reference.

In some embodiments, the composition further comprises water as a component. The amount of water present in the composition may vary, but is generally less than about 15%, such as from about 5 to about 15, or from about 9 to about 11% by weight, based on the weight of the composition. Accordingly, in some embodiments, water is combined with the dry ingredients, alone or in admixture with other liquid ingredients (e.g., humectants).

The resulting composition is then enclosed in a pouch as described herein above. In some embodiments, the composition is introduced into the pouch material in the form of a particulate material (e.g., as a dry powder). Various manufacturing apparati and methods can be used to create a pouched product described herein. For example, US Publication No. 2012/0055493 to Novak, III et al., previously incorporated by reference in its entirety, relates to an apparatus and process for providing pouch material formed into a tube for use in the manufacture of oral products. Similar apparatuses that incorporate equipment for supplying a continuous supply of a pouch material (e.g., a pouch processing unit adapted to supply a pouch material to a continuous tube forming unit for forming a continuous tubular member from the pouch material) can be used to create a pouched product described herein. Representative equipment for forming such a continuous tube of pouch material is disclosed, for example, in U.S. Patent Application Publication No. US 2010/0101588 to Boldrini et al., which is incorporated herein by reference in its entirety. The apparatus further includes equipment for supplying pouched material to the continuous tubular member such that, when the continuous tubular member is subdivided and sealed into discrete pouch portions, each pouch portion includes a charge of a composition adapted for oral use. Representative equipment for supplying the filler material is disclosed, for example, in U.S. Patent Application Publication No. US 2010/0018539 to Brinkley, which is incorporated herein by reference in its entirety. In some instances, the apparatus may include a subdividing unit for subdividing the continuous tubular member into individual pouch portions and, once subdivided into the individual pouch portions, may also include a sealing unit for sealing at least one of the ends of each pouch portion. In other instances, the continuous tubular member may be sealed into individual pouch portions with a sealing unit and then, once the individual pouch portions are sealed, the continuous tubular member may be subdivided into discrete individual pouch portions by a subdividing unit subdividing the continuous tubular member between the sealed ends of serially disposed pouch portions. Still in other instances, sealing (closing) of the individual pouch portions of the continuous tubular member may occur substantially concurrently with the subdivision thereof, using a closing and dividing unit. It is noted that in some embodiments of the present disclosure wherein a low melting point binder material is used, the temperature required for sealing the seams of the pouched product can be less than the temperature required in conventional processes associated with conventional binder materials.

The pouched product may be contacted with water to provide the desired moisture content of the final pouched product. The moisture content (e.g., water content) of the oral product, prior to use by a consumer of the oral product, may vary according to the desired properties. Typically, the oral product, prior to insertion into the mouth of the user, is from about 5 to about 60% by weight of water, for example, from about 5 to about 15%, from about 15 to about 25%, from about 25 to about 40%, or from about 40 to about 60% water by weight, based on the total weight of the oral product. In some embodiments, the pouched product has a water content from about 30 to about 45% by weight. In some embodiments, the desired amount of water is sprayed or dripped onto the pouch.

Many modifications and other embodiments of the disclosure will come to mind to one skilled in the art to which this disclosure pertains having the benefit of the teachings presented in the foregoing description. Therefore, it is to be understood that the disclosure is not to be limited to the specific embodiments disclosed and that modifications and other embodiments are intended to be included within the scope of the appended claims. Although specific terms are employed herein, they are used in a generic and descriptive sense only and not for purposes of limitation.

### EXAMPLES

Aspects of the present disclosure are more fully illustrated by the following examples, which are set forth to illustrate certain aspects of the present disclosure and are not to be construed as limiting thereof.

### Determination of pH

A sample (5.0±0.1 grams) of the relevant material (pouched product, SEDS component, or composition as disclosed herein) was placed in a specimen container. Deionized water (50±1 mL) was added, and the mixture stirred with a magnetic stir bar for 15 minutes. The pH was measured on a calibrated Orion Model 3 Combination Electrode and Meter.

### Particle size measurements

Particle size measurements for the droplets in the various emulsions were made on a Malvern Zetasizer Nano ZS. The sample material properties were set to a refractive index of ca. 1.395 and absorption of 0.010 (SEDS system properties measured on a standard refractometer) and default "water" setting as the dispersant. Samples were prepared by dispersing the SEDS component or composition in water at a ratio of about 100: 1 water to sample (1% SEDS) in 50 mL conical centrifuge tubes. Samples were agitated to achieve homogeneity and 2-5 mL aliquots were dispensed into 12 mm square polystyrene cuvettes. The samples were allowed to sit without disturbance in the instrument for 120 seconds. Measurements were taken in three sets of 12 averaged scans. The average of the three sets was recorded as the particle (micelle) size.

### Example 1. Self-Emulsifying Delivery System 1 (SEDS1)

A self-emulsifying delivery system (SEDS) was prepared using the components and quantities provided in **Table 2.** The linoleic acid monoglyceryl ester (Maisine^{®}; solubilizer), surfactants (Kolliphor^{®} EL and Labrafil^{®} 1944), a flavor, and an antioxidant (tocopherols) were combined to provide a pre-emulsion (SEDS).

**Table 2. SEDS components and quantities**

| **Component** | **Percent by weight** |
|---|---|
| polyethoxylated castor oil (Kolliphor^{®} EL) | 50 |
| linoleic acid monoglyceryl ester (Maisine^{®}) | 21 |
| PEG-6 mono- and diesters of oleic acid (Labrafil^{®} 1944) | 21 |
| tocopherols | 0.5 |
| flavor | 7.5 |

### Example 2. Self-Emulsifying Delivery System 2 (SEDS2)

A self-emulsifying delivery system was prepared using the components and quantities provided in **Table 3.** The solubilizer (Peceol^{™}), a surfactant (Kolliphor^{®} EL), a lipid (olive oil), a flavor, and an antioxidant were combined to provide a pre-emulsion (SEDS).

**Table 3. SEDS components and quantities**

| **Component** | **Percent bv weight** |
|---|---|
| polyethoxylated castor oil (Kolliphor^{®} EL) | 50 |
| Olive oil | 21 |
| glyceryl monooleate (Peceol^{™}) | 21 |
| tocopherols | 0.5 |
| flavor | 7.5 |

### Example 3. Pouched SEDS1

A pouched product comprising the SEDS (pre-emulsion) of Example 1 was prepared using the formulation provided in **Table 4.** The SEDS was adsorbed on the carrier material (e.g., silica) and the nicotine was adsorbed on the microcrystalline cellulose. The SEDS/silica and mcc/nicotine components were combined with the remaining ingredients to form the composition, which was placed in pouches (365 mg of composition per pouch; 6 mg nicotine content). The pouches were sprayed with water to provide a 35% moisture content.

**Table 4. Pouched product composition components and quantities**

| **Component** | **Percent by weight** |
|---|---|
| SEDS1 | 1.5-3.5 |
| microcrystalline cellulose | 32-48 |
| sodium chloride | 2-3 |
| 5.6% nicotine solution | 20-40 |
| water | 9-11 |
| Carrier material (e.g., silica) | 6-8 |
| glycerin | 7-9 |
| propylene glycol | 1-1.5 |
| sodium bicarbonate | 0.2-0.3 |
| sweetener | 1-3 |

### Example 4. Pouched SEDS2

A pouched product was prepared as in Example 3, but using the SEDS (pre-emulsion) of Example 2.

## Claims

1. A pouched oral product comprising a pouch and a composition enclosed therein, the composition comprising a self-emulsifying delivery system (SEDS) component, the SEDS component comprising:
a flavoring agent, an active ingredient, or a combination thereof;
at least one solubilizer;
at least one emulsifying agent; and
optionally, an antioxidant, a lipid component, or both an antioxidant and a lipid component; and
optionally, wherein the SEDS component provides micelles comprising the flavoring agent, the active ingredient, or both, when dispersed in water at a ratio of about 100: 1 water to SEDS component by weight.

2. The oral product of claim 1, wherein the at least one solubilizer comprises a glycerol monoester of a fatty acid.

3. The oral product of claim 1 or 2, wherein the at least one emulsifying agent has a hydrophilic-lipophilic balance (HLB) value in a range from about 3 to about 5.

4. The oral product of any one of claims 1-3, wherein the at least one emulsifying agent comprises polyethoxylated castor oil, a polyethylene glycol monoester of a fatty acid, a polyethylene glycol diester of a fatty acid, or a combination thereof.

5. The oral product of claim 1, wherein one or more of the following applies:
the active ingredient comprises one or more botanical materials, stimulants, nicotine components, amino acids, vitamins, cannabinoids, cannabimimetics, terpenes, nutraceuticals, pharmaceutical agents, or combinations thereof;
the flavoring agent comprises one or more aldehydes, ketones, esters, terpenes, terpenoids, or a combination thereof;
the antioxidant is a tocopherol, BHT, a TBHQ, vitamin E or a derivative thereof, citric acid, a fatty acid ester of vitamin C, or a combination thereof;
the lipid component is a natural, food-grade oil; optionally, wherein the lipid component is olive oil.

6. The oral product of any one of claims 1-5, wherein the SEDS component comprises:
from about 40 to about 60% by weight of polyethoxylated castor oil;
from about 17 to about 25% by weight of linoleic acid monoglyceryl ester;
from about 17 to about 25% by weight of a mixture of polyethylene glycol 6 mono- and diesters of oleic acid;
from about 0.4 to about 0.6% by weight of tocopherols,
wherein each amount by weight is based on the total weight of the SEDS component.

7. The oral product of any one of claims 1-6, wherein the SEDS component is at least partially in the form of a pre-emulsion comprising micelles having an average particle size in a range from about 100 to about 300 nm.

8. The oral product of any one of claims 1-7, wherein the composition further comprises:
a filler;
a carrier material;
optionally, a further active ingredient, a further flavoring agent, or a combination thereof; and
optionally, one or more sweeteners, one or more humectants, one or more binding agents, one or more salts, one or more buffering agents, or a combination thereof.

9. The oral product of any one of claims 1-8, wherein one or more of the following applies:
the oral product comprises the SEDS component in an amount by weight from about 2 to about 5%, based on the total weight of the oral product;
the SEDS component is adsorbed in or on the carrier material; optionally, wherein the carrier material is silica;
the filler comprises a cellulose material, optionally, wherein the cellulose material comprises microcrystalline cellulose.

10. The oral product of claim 8 or 9, comprising:
from about 10 to about 50% of the filler; and
from about 5 to about 60% by weight of water, based on the total weight of the oral product.

11. The oral product of any one of claims 8-10, wherein the further active ingredient comprises a nicotine component, botanical materials, stimulants, amino acids, vitamins, antioxidants, cannabinoids, cannabimimetics, terpenes, nutraceuticals, pharmaceutical agents, or combinations thereof; optionally, wherein the further active ingredient comprises a nicotine component.

12. The oral product of any one of claims 8-11, wherein the further active ingredient comprises a nicotine component, and wherein the composition further comprises one or more organic acids, an alkali metal salt of the one or more organic acids, or both.

13. The oral product of claim 12, wherein one or more of the following applies:
the one or more organic acids or alkali metal salt thereof is present in the composition in an amount by weight of from about 0.1 to about 10%, or from about 0.1 to about 0.5%, based on the total weight of the composition;
the one or more organic acids is citric acid, malic acid, tartaric acid, octanoic acid, benzoic acid, a toluic acid, salicylic acid, or a combination thereof;
at least a portion of the organic acid, the alkali metal salt of the organic acid, or both are associated with at least a portion of the nicotine component in the form of an ion pair.

14. A method of preparing a pouched oral product comprising a self-emulsifying delivery system (SEDS) component comprising at least one solubilizer, at least one emulsifying agent, and optionally, an antioxidant, a lipid component, or both an antioxidant and a lipid component, the method comprising:
combining at least one solubilizer, at least one emulsifying agent, and optionally, an antioxidant, a lipid component, or both an antioxidant and a lipid component, to form the SEDS component;
contacting the SEDS component with a carrier material to form an adsorbed SEDS component;
combining the adsorbed SEDS component with:
a filler;
an active ingredient, a flavoring agent, or a combination thereof; and optionally, one or more sweeteners, one or more humectants, one or more binding agents, one or more salts, one or more buffering agents, or a combination thereof, to form a composition configured for oral use;
enclosing the composition in a pouch; and
adding an amount of water to the pouch.

15. The method of claim 14, wherein one or more of the following applies:
the at least one solubilizer comprises a glycerol monoester of a fatty acid;
the at least one emulsifying agent has a hydrophilic-lipophilic balance (HLB) value in a range from about 3 to about 5;
the at least one emulsifying agent comprises polyethoxylated castor oil, a polyethylene glycol monoester of a fatty acid, a polyethylene glycol diester of a fatty acid, or a combination thereof;
the antioxidant is a tocopherol, BHT, a TBHQ, vitamin E or a derivative thereof, citric acid, a fatty acid ester of vitamin C, or a combination thereof;
the lipid component is a natural, food-grade oil; optionally, wherein the lipid component is olive oil;
the filler comprises a cellulose material, optionally, wherein the cellulose material comprises microcrystalline cellulose; and
the carrier material is silica.
